(19) Europäisches Patentamt
European Patent Office
Office européen des brevets

(11) Veröffentlichungsnummer: **0 145 664**
**B1**

(12) **EUROPÄISCHE PATENTSCHRIFT**

(45) Veröffentlichungstag der Patentschrift: **27.04.88**

(51) Int. Cl.⁴: **C 07 D 401/12,** A 01 N 47/36 // C07D213/70

(21) Anmeldenummer: **84810590.4**

(22) Anmeldetag: **03.12.84**

(54) **Herbizide Sulfonylharnstoffe.**

(30) Priorität: **08.12.83 CH 6574/83**

(43) Veröffentlichungstag der Anmeldung: **19.06.85 Patentblatt 85/25**

(45) Bekanntmachung des Hinweises auf die Patenterteilung: **27.04.88 Patentblatt 88/17**

(84) Benannte Vertragsstaaten: **AT BE CH DE FR GB IT LI NL**

(56) Entgegenhaltungen:
**EP-A-0 030 433**
**EP-A-0 035 893**
**EP-A-0 094 790**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Meyer, Willy, Talweg 49, CH- 4125 Riehen (CH)**
Erfinder: **Föry, Werner, Dr., Benkenstrasse 65, CH- 4054 Basel (CH)**

**EP 0 145 664 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue, herzizid wirksame und pflanzenwuchsregulierende substituierte N-Pyridinylsufonyl-N'-pyrimidinyl und triazinyl-harnstoffe, Verfahren zu ihrer Herstellung, sie als Wirkstoffe enthaltende Mittel sowie deren Verwendung zum Bekämpfen von Unkräutern, vor allem selektiv in Nutzpflanzenkulturen oder zum Regulieren und Hemmen des Pflanzenwachstums. Darüberhinaus betrifft die Erfindung auch als Zwischenprodukte hergestellte neue substituierte Pyridinylsulfonamide.

Die erfindungsgemässen substituierten N-Pyridinylsulfonyl-N'-pyrimidinyl- und -triazinyl-harnstoffe entsprechen der Formel I

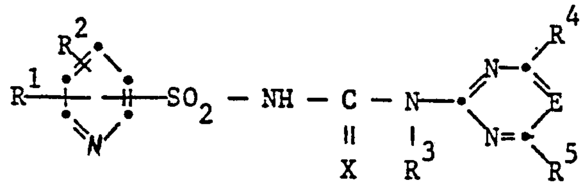

(I)

worin

$R^1$ einen Rest 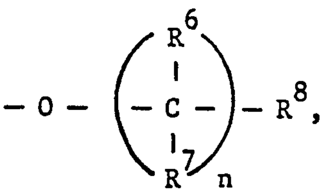

$R^2$ Wasserstoff, Halogen, Nitro, $C_1$-$C_3$-Alkyl, $C_1$-$C_3$-Alkoxy oder -$COOR^9$,
$R^3$ Wasserstoff, $C_1$-$C_3$-Alkyl oder $C_1$-$C_3$-Alkoxy,
$R^4$ und $R^5$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_2$-$C_4$-Alkoxyalkyl, $C_3$-$C_6$-Cycloalkyl oder - $NR^{10}R^{11}$,
$R^6$ Wasserstoff oder $C_1$-$C_3$-Alkyl,
$R^7$ Wasserstoff oder Methyl,
$R^8$ unsubstituiertes oder durch $C_1$-$C_3$-Alkyl oder Halogen substituiertes $C_4$-$C_6$-Cycloalkanoyl; unsubstituiertes oder ein- oder mehrfach durch $C_1$-$C_3$-Alkyl, $C_1$-$C_3$-Alkoxy, Halogen oder Cyan substituiertes $C_3$-$C_6$-Cycloalkyl; oder unsubstituiertes oder durch $C_1$-$C_3$-Alkyl, Halogen oder Cyan substituiertes $C_5$-$C_6$-Cycloalkenyl,
$R^9$ $C_1$-$C_3$-Alkyl oder Allyl,
$R^{10}$ und $R^{11}$ unabhängig voneinander Wasserstoff oder $C_1$-$C_4$-Alkyl,
n die Zahl Null oder eins,
X Sauerstoff oder Schwefel und
E Stickstoff oder die Methinbrücke sowie die Salze dieser Verbindungen.

Harnstoffverbindungen Triazinverbindungen und Pyrimidinverbindungen mit herbizider Wirkung sind allgemein bekannt. Kürzlich wurden Sulfonylharnstoffverbindungen mit herbizider und pflanzenwuchsregulierender Wirkung, beispielsweise in den europäischen Patentanmeldung 30433, 35893, 44211, 44807, 85028 und 94790 beschrieben.

In den Definitionen ist unter Alkyl geradkettiges oder verzweigtes Alkyl zu verstehen; z. B: Methyl, Äthyl, n-Propyl oder i-Propyl.

Unter Alkoxy ist zu verstehen: Methoxy, Äthoxy, n-Propoxy oder i-Propoxy, insbesondere aber Methoxy, Äthoxy oder i-Propoxy.

Beispiele für Alkylthio sind Methylthio, Äthylthio, n-Propylthio oder i-Propylthio, insbesondere aber Methylthio und Äthylthio.

Unter Halogen selbst in den Definitionen sowie Halogen als Teil in Halogenalkoxy Halogenalkyl oder Halogenalkylthio sind Fluor, Chlor und Brom, vorzugsweise jedoch Fluor und Chlor zu verstehen.

Definitionsgemässe Alkoxyalkylwerte sind beispielsweise Methoxymethyl, Aethoxymethyl, Methoxyäthyl und Äthoxyäthyl.

Unter den in $R^8$ definierten Carbocyclen sind im Rahmen der vorliegenden Anmeldung zum Beispiel folgende Reste zu verstehen: Cyclopropyl, Cyclobutyl, Cyclopentyl, Cyclohexyl, 2-Oxocyclobutyl, 3-Oxocyclobutyl, 2-Oxocyclopentyl, 3-Oxocyclopentyl, 2-Oxocyclohexyl, 3-Oxocyclohexyl, 4-Oxocyclohexyl, 1-Cyclopentenyl, 2-Cyclopentenyl, 3-Cyclopentenyl, 1-Cyclohexenyl, 2-Cyclohexenyl oder 3-Cyclohexenyl. Bevorzugte Carbocyclentypen sind Cycloalkylreste, insbesondere Ciclopentyl und Cyclohexyl.

Die definitionsgemässen Carbocyclen $R^8$ können an das Sauerstoffatom direkt oder über ein weiteres Kohlenstoffatom gebunden sein. Bevorzugt ist die direkte Bindung, d.h. n steht für die Zahl Null.

Halogenalkyl als Teil eines anderen Substituenten wie Halogenalkoxy oder Halogenalkylthio steht in der

Regel für Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Die Erfindung umfasst ebenfalls die Salze, die die Verbindungen der Formel I mit Aminen, Alkali- und Erdalkalimetallbasen oder quaternären Amoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Äthylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Äthyl-, Propyl-, Diäthyl- oder Triäthylamin, vor allem aber iso-Propylamin, Diäthanolamin und 1,4-Diazabicyclo[2.2.2]octan.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Unter den Verbindungen der Formel I sind diejenigen bevorzugt, in denen entweder

a) X Sauerstoff ist oder

b) $R^2$ Wasserstoff ist oder

c) $R^3$ Wasserstoff ist oder

d) $R^4$ und R unabhängig voneinander für Halogen, $C_1$-$C_3$-Alkyl, $C_1$-$C_3$-Alkoxy, Di-$C_1$-$C_3$-alkylamino oder $C_1$-$C_3$-Halogenalkoxy stehen und zusammen höchstens 4 Kohlenstoffatome enthalten oder

e) $R^1$ für -O-$R^8$ steht oder

f) $R^1$ für -O-CH$R^7$ -Cyclopropyl steht.

Unter den Verbindungen der Untergruppe e) sind besonders solche Verbindungen bevorzugt, indem $R^8$ fur $C_3$-$C_6$-Cycloalkyl, insbesondere aber für Cyclopentyl oder Cyclohexyl steht.

Als weitere hervorzuhebende Untergruppen von Verbindungen der Formel I sind diejenigen zu nennen, in denen X für Sauerstoff steht, $R^2$ und $R^3$ Wasserstoff bedeuten, $R^4$ und $R^5$ unabhängig voneinander für Halogen, $C_1$-$C_3$-Alkyl, $C_1$-$C_3$-Alkoxy, Di-$C_1$-$C_3$-Alkylamino oder $C_1$-$C_3$-Halogenalkoxy stehen und zusammen höchstens 4 Kohlenstoffatome enthalten und $R^1$ für einen Rest -O-$R^8$ steht; und besoders diejenigen, in denen X für Sauerstoff steht, $R^2$ und $R^3$ Wasserstoff bedeuten, $R^4$ und $R^5$ unabhängig voneinander für $C_1$-$C_3$-Alkyl, Halogen, $C_1$-$C_3$-Alkoxy, Di-$C_1$-$C_3$-alkylamino oder $C_1$-$C_3$-Halogenalkoxy stehen und zusammen höchstens 4 Kohlenstoffatome enthalten und $R^1$ für $C_3$-$C_6$-Cycloalkoxy, insbesondere aber Cyclopentyloxy oder Cyclohexyloxy steht.

Als bevorzugte Einzelverbindungen der Formel I sind zu nennen:

N-(2-Cyclopentyloxypyridin-3-ylsulfonyl)-N'-(4-methoxy-6-methyl-1,3,5-triazin-2-yl)-harnstoff und

N-(2-Cyclopropylmethoxypyridin-3-ylsulfonyl)-N'-(4-methoxy-6-methyl-1,3,5-triazin-2-yl)-harnstoff.

Die Herstellung der Verbindungen der Formel I erfolgt im allgemeinen nach den folgenden Methoden.

Nach einem ersten Verfahren werden die Verbindungen der Formel I erhalten, indem man ein substituiertes Pyridinylsulfonamid der Formel II

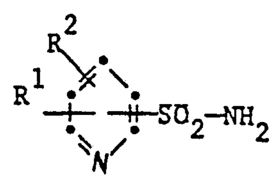

(II),

worin $R^1$ und $R^2$ die unter Formel I gegebene Bedeutung haben, in Gegenwart einer Base mit einem N-Pyrimidinyl-oder -triazinylcarbamat der Formel III

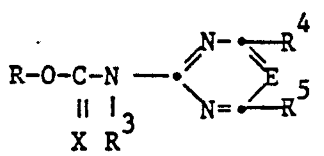

(III)

worin E, $R^3$, $R^4$, $R^5$ und X die unter Formel I gegebene Bedeutung haben und R für Phenyl, Alkyl oder substituiertes Phenyl steht, umsetzt.

Nach einem zweiten Verfahren gelangt man zu Verbindungen der Formel I, indem man ein substituiertes N-Pyridinylsulfonylcarbamat der Formel IV

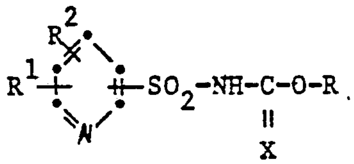

(IV),

worin $R^1$, $R^2$ und x die unter Formel I gegebene Bedeutung haben und R für Phenyl, Alkyl oder substituiertes Phenyl steht, mit einem Aminopyrimidin oder -triazin der Formel V

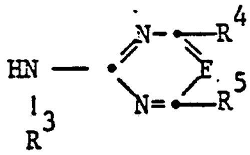

(V),

worin E, $R^3$, $R^4$ und $R^5$ die unter Formel I gegebene Bedeutung haben, umsetzt.

Schliesslich kann man die Verbindungen der Formel I auch erhalten, indem man ein Sulfonylisocyanat der Formel VI

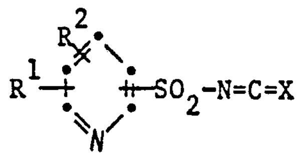

(VI),

worin $R^1$, $R^2$ und X die unter Formel I gegebene Bedeutung haben, mit einem Aminopyrimidin oder -triazin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze überführt werden. Dieses geschieht beispielsweise durch Umsetzen mit der äquimolaren Menge Base und Verdampfen des Lösungsmittels.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Methylenchlorid, Chloroform, Tetrachlorkohlenstoff, oder Chlorbenzol, Äther wie Diäthyläther, Äthylenglykoldimethyläther, Diäthylenglykoldimethyläther, Tetrahydrofuran oder Dioxan, Nirile wie Acetonitril oder Propionitril, Amide wie Dimethylformamid, Diäthylformamid oder N-Methylpyrrolidinon. Die Reaktionstemperaturen liegen vorzugsweise zwischen -20° und +120°C. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triäthylamin, Chinuclidin, 1,4-Diazabicyclo-[2,2,2]-octan. 1,5-Diazabicyclo[4,3,0]non-5-en oder 1,8-Diazabicyclo[5,4,0]undec-7-en geeignet. Als Basen können aber auch anorganische Basen wie Hydride wie Natrium- oder Calciumhydrid, Hydroxyide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Äther, aromatischen Kohlenwasserstoffen oder chlorieren Kohlenwasserstoffen gereinigt werden.

Die Zwischenprodukte der Formeln II, IV und VI sind neu und wurden speziell für die Synthese der Verbindungen der Formel I entwickelt. Sie bilden daher einen Bestandteil der vorliegenden Erfindung.

Die neuen Zwischenprodukte der Formel II werden nach an sich bekannten Verfahren hergestellt. So erhält man die Verbindungen der Formel II beispielsweise, indem man ein Halogenpyridinylsulfonamid der Formel VII

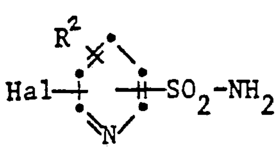

(VII),

worin $R^2$ die unter Formel I gegebene Bedeutung hat und Hal für Halogen, vorzugsweise Fluor oder Chlor steht, in Gegenwart einer Base in einem polaren aprotischen Lösungsmittel mit einem Alkohol der Formel VIII

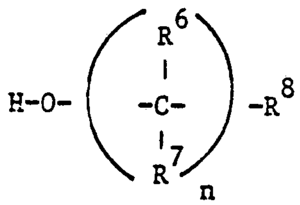 (VIII),

worin $R^6$, $R^7$, n und $R^8$ die unter Formel I gegebene Bedeutung haben, umsetzt.

Weiter erhält man die neuen Verbindungen der Formel II, indem man Amine der Formel IX

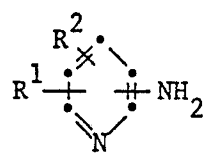 (IX),

worin $R^1$ und $R^2$ die unter Formel I gegebene Bedeutung haben, diazotiert und die Diazogruppe mit Schwefeldioxid in Gegenwart eines Katalysators wie Kupferchlorid in Salzsäure oder Essigsäure austauscht und das entstandene Pyridinylsulfonylchlorid der Formel X

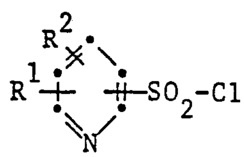 (X),

worin $R^1$ und $R^2$ die unter Formel I angegebene Bedeutung haben, mit Ammoniumhydroxid-Lösung umsetzt. Die als Ausgangsprodukte verwendeten entsprechenden Amine sind bekannt oder nach bekannten Methoden herstellbar.

Ebenso kann man die neuen Verbindungen der Formel II erhalten, indem man eine Pyridinylsulfonsäure der Formel XI

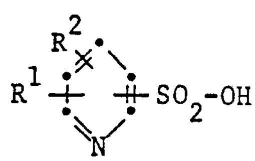 (XI),

worin $R^1$ und $R^2$ die unter Formel I gegebene Bedeutung haben, durch Behandeln mit einem Chlorierungsmittel wie $PCl_5$, $POCl_3$ $COCl_2$ oder $SOCl_2$ in das entsprechende Pyridinylsulfonylchlorid der Formel X überführt und dieses mit Ammoniumhydroxid-Lösung umsetzt.

Weiter kann man die Verbindungen der Formel IIa erhalten, indem man einen Benzylthioäther der Formel XII

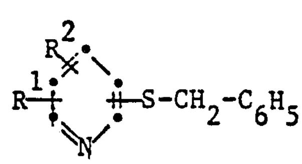 (XII)

worin $R^1$ und $R^2$ die unter Formel I gegebene Bedeutung haben, mit Chlor behandelt und das entstandene Pyridinylsulfonylchlorid der Formel X mit Ammoniumhydroxid-Lösung umsetzt.

Die ebenfalls neuen Pyridinylsulfonylisocyanate der Formel VI können beispielsweise durch Umsetzungen der Sulfonamide der Formel II mit Phosgen in Anwesenheit von Butylisocyanat in einem chlorierten Kohlenwasserstoff als Lösungsmittel, bei Rückflusstemperatur erhalten werden. Ähnliche Darstellungen sind in "Neuere Methoden der Präparativen organischen Chemie", Band VI, 211-229, Verlag Chemie, Weinheim. 1970, beschreiben.

Die Isothiocyanate der Formel VI werden durch Behandlung der Sulfonamide der Formel II mit Schwefelkohlenstoff und Kaliumhydroxid und anschliessende Umsetzung des Dikaliumsalzes mit Phosgen

erhalten. Solche Verfahren sind in Arch. Pharm. 299, 174 (1966) beschrieben.

Die N-Pyridinylsulfonylcarbamate der Formel IV werden durch Umsetzung der Sulfonamide der Formel II mit einem Kohlensäureester in Gegenwart einer Base erhalten. Ähnliche Verfahren sind in der japanischen Patentschrift 61 169 erwähnt.

Die als Ausgangsmaterialien verwendeten Aminopyrimidine und -triazine der Formel V sowie entsprechende Carbamate der Formel III sind entweder seit langem bekannt oder in der europäischen Patentanmeldung Nr. 70804 beschrieben oder sie lassen sich nach bekannten Methoden aus dort beschriebenen Verbindungen erhalten.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Äther, aromatischen Kohlenwasserstoffen oder chlorierten Kohlenwasserstoffen gereinigt werden.

Die Wirkstoffe der Formel I sind stabile Verbindungen. Ihre Handhabung bedarf keine vorsorglicher Massnahmen.

Die Verbindungen der Formeln VII, VIII, IX, X, XI und XII sind bekannt oder können analog zu bekannten Verfahren hergestellt werden.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt. So können z. B. die in der Landwirtschaft in tropischen Gegenden häufig als "cover crops" (Bodenbedecker) angepflanzte Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzgut die Fraktionen $C_8$ bis $C_{12}$, wie z. B. Xylolgemische oder substituierte Naphtahline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, sas nicht sorptive Trägermaterialien z. B. Calcit oder Sand in Frage. Darüberhinaus kann eine Vielzahl von vorgranulierten Materialien anorganischer oder organischer Natur

wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschafen in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Öl- oder Stearinsäure, oder von natürlichen Fettsäuregemischen, die z. B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl-oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1981;

H. Stache, "Tensid-Taschenbuch", 2. Aufl., C. Hanser Verlag, München, Wien, 1981;

M. and J. Ash. "Encyclopedia of Surfactants", Vol. I-III, Chemical Publishing Co., New York, 1980-1981.

Die herbiziden Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 % insbesondere 0,1 bis 25 % eines Tensides.

Insbesondere setzen sich bevorzugte Formulierungen folgendermassen zusammen: (% = Gewichtsprozent).

**Emulgierbare Konzentrate**:

| | |
|---|---|
| Aktiver Wirkstoff: | 1 bis 20 %, bevorzugt 5 bis 10 % |
| oberflächenaktive Mittel: | 5 bis 30 %, vorzugsweise 10 bis 20 % |
| flüssiges Trägermittel: | 50 bis 94 %, vorzugsweise 70 bis 85 % |

**Stäube:**

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

**Suspensions-Konzentrate:**

| | |
|---|---|
| Aktiver Wirkstoff: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 % |

**Benetzbares Pulver:**

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 % |

**Granulate:**

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Während als Handelsware eher konzentriete Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

**Beispiel H 1**: N-(2-Cyclopentyloxypyridin-3-ylsulfonyl)-N'-(4-methoxy-6-methyl-1,3,5-triazin-2-yl)-harnstoff (Verb. Nr. 2.1)

a) 2-Cyclopentyloxypyridin-3-ylsulfonamid,

10,9 g einer 55 %-igen Natriumhydriddispersion werden in 40 ml Dimethylformamid suspendiert und tropfenweise mit 13,6 ml Cyclopentanol versetzt. Anschliessend lässt man eine Lösung von 19,24 g 2-Chlorpyridin-3-ylsulfonamid in 40 ml Dimethylformamid zutropfen und rührt die Reaktionsmischung für zwei Stunden bei 35-40° C. Durch Aufnehmen des Reaktionsgemisches in einer Mischung aus 250 ml Äthylacetat, 250 ml Eiswasser und 125 ml 1N Salzsäure, dreimalige Extraktion mit je 100 ml Äthylacetat, viermaliges Waschen der vereinigten organischen Phasen mit je 100 ml Wasser, Trocknen und Eindampfen der organischen Lösung erhält man einen öligen Rückstand, der nach Verreiben mit 50 ml Äther kristallisiert. Man erhält so 19,64 g 2-Cyclopentyloxypyridin-3-ylsulfonamid, Smp. 134-135° C.

b) Einer Mischung von 4,84 g 2-Cyclopentyloxypyridin-3-ylsulfonamid und 3,13 ml 1,8-Diazabicyclo[5.4.0]undec-7-en in 50 ml absolutem Dioxan setzt man bei einer Temperatur zwischen 20° und 25° C 5,46 g N-(4-Methoxy-6-methyl-1,3,5-triazin-2-yl)-phenylcarbamat zu. Die Mischung wird für weitere 0,5 Std. gerührt, vom Niederschlag abgetrennt und eingedampft. Der Rückstand wird mit 10 ml 2N Salzsäure verrieben, und das entstehende Kristallisat isoliert, mit Wasser und Äther gewaschen und getrocknet. Man erhält so 7,79 g N-(2-Cyclo-pentyloxypyridin-3-ylsulfonyl)-N'-(4-methoxy-6-methyl-1,3,5-triazin-2-yl)-harnstoff, Smp. 170-172° C.

**Beispiel H 2**: N-(2-Cyclopropylmethoxypyridin-3 - ylsulfonyl)-N'-(4-methoxy-6-methyl-1,3,5-triazin-2-yl)-harnstoff (Verb. Nr. 2.60)

a) 2-Cyclopropylmethoxypyridin-3-ylsulfonamid.

11,99 g einer 55 %-igen Natriumhydriddispersion werden in 40 ml Dimethylformamid suspendiert und bei 10° C unter einer Stickstoffatmosphäre tropfenweise mit 13,04 ml Cyclopropylmethanol versetzt. Die Mischung wird für 15 Minuten gerührt und anschliessend tropfenweise mit einer Lösung von 21,16 g 2-Chlorpyridin-3-ylsulfonamid in 40 ml Dimethylformamid versetzt. Nachdem das Reaktionsgemisch für 30 Minuten bei einer Temperatur von 35-40° C gerührt worden ist, wird es in einer Mischung von 275 ml Äthylacetat, 275 ml Eiswasser und 137,5 ml 2N Salzsäure aufgenommen. Die organische Phase wird abgetrennt, die wässrige Lösung dreimal mit je 100 ml Äthylacetat extrahiert. Die vereinigten organischen Phasen werden viermal mit je 100 ml Wasser gewaschen, getrocknet, eingedampft und mit Äther/Hexan (2 : 1) verrieben. Das anfallende Kristallisat wird abgetrennt und getrocknet. Man erhält 22,25 g 2-Cyclopropylmethoxypyridin-3-ylsulfonamid, Smp. 121-122° C.

b) 4,56 g 2-Cyclopropylmethoxypyridin-3-ylsulfonamid und 3,13 ml 1,8 Diazabicyclo[5.4.0]undec-7-en werden in 50 ml absolutem Dioxan suspendiert, und mit 5,46 g N-(4-Methoxy-6-methyl-1,3,5-triazin-2-yl)-phenylcarbamat versetzt. Die Mischung wird für 20 Minuten gerührt und eingedampft. Der Rückstand wird mit

10 ml 2N Salzsäure verrieben, der ausgefallene Festkörper abgetrennt und mit Wasser und Äther gewaschen. Man erhält 6, 90 g N-(2-Cyclopropylmethoxypyridin-3-ylsulfonyl)-N'-(4-methoxy-6-methyl-1,3,5-triazin-2-yl)-harnstoff, Smp. 165-167° C.

In analoger Weise werden die in den angeschlossenen Tabellen aufgelisteten Zwischen- und Endprodukte hergestellt.

**Tabelle 1**:

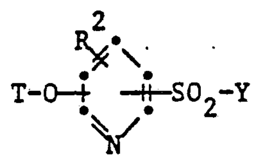

| Verb. Nr. | $R^2$ | T | Stellung-O-T Rest | Y | Stellung $-SO_2-Y$ Rest | Phys. Daten |
|---|---|---|---|---|---|---|
| 1.1 | H | Cyclohexyl | 2 | $NH_2$ | 3 | Smp. 134-135° C |
| 1.2 | H | Cyclopropylmethyl | 2 | $NH_2$ | 3 | Smp.146-147° C |
| 1.3 | H | Cyclopropylmethyl | 2 | $NH_2$ | 3 | Smp. 121-122° C |
| 1.4 | H | 1-Methylcyclopropyl | 2 | $NH_2$ | 3 | |
| 1.5 | H | Cyclopentylmethyl | 2 | $NH_2$ | 3 | |
| 1.6 | H | 2-Chlorcyclopentyl | 2 | $NH_2$ | 3 | |
| 1.7 | H | Cyclopentyl | 3 | $NH_2$ | 2 | |

**Tabelle 2**:

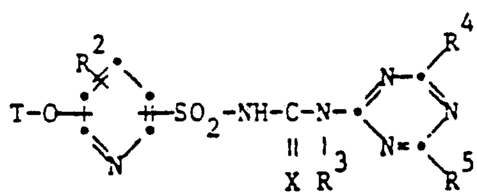

| Verb. Nr. | T | Stellung -O-T Rest | $R^2$ | $R^3$ | $R^4$ | $R^5$ | X | Stellung $-SO_2$-Rest | Smp.[° C] |
|---|---|---|---|---|---|---|---|---|---|
| 2.1 | cyclopentyl | 2 | H | H | $CH_3$ | $OCH_3$ | O | 3 | 170-172 |
| 2.2 | Cyclopentyl | 2 | H | H | $OCH_3$ | $OCH_3$ | O | 3 | 192-194 |
| 2.3 | Cyclopentyl | 2 | H | H | Cl | $OCH_3$ | O | 3 | |
| 2.4 | Cyclopentyl | 2 | H | H | $CH_3$ | $OC_2H_5$ | O | 3 | |
| 2.5 | Cyclopentyl | 2 | H | H | $OCH_3$ | $OC_2H_5$ | O | 3 | |
| 2.6 | Cyclopentyl | 2 | H | H | $CH_3$ | $OCH(CH_3)_2$ | O | 3 | |
| 2.7 | Cyclopentyl | 2 | H | H | $OCH_3$ | $C_2H_5$ | O | 3 | |
| 2.8 | Cyclopentyl | 2 | H | H | $CH_3$ | $C_2H_5$ | O | 3 | |
| 2.9 | Cyclopentyl | 2 | H | H | $N(CH_3)_2$ | $OCH_2CF_3$ | O | 3 | |
| 2.10 | Cyclopentyl | 2 | H | H | $CH_3$ | $SCHF_2$ | O | 3 | |
| 2.11 | Cyclohexyl | 2 | H | H | $CH_3$ | $OCH_3$ | O | 3 | 154-155 |
| 2.12 | Cyclohexyl | 2 | H | H | $OCH_3$ | $OCH_3$ | O | 3 | 170-172 |
| 2.13 | Cyclohexyl | 2 | H | H | $C_2H_5$ | $OCH_3$ | O | 3 | |
| 2.14 | Cyclohexyl | 2 | H | H | $CH_3$ | $OC_2H_5$ | O | 3 | |
| 2.15 | Cyclohexyl | 2 | H | H | $OCH_3$ | $OCH_2CF_3$ | O | 3 | |
| 2.16 | Cyclobutyl | 2 | H | H | $CH_3$ | $OCH_3$ | O | 3 | |
| 2.17 | Cyclobutyl | 2 | H | H | $OCH_3$ | $OCH_3$ | O | 3 | |
| 2.18 | Cyclobutyl | 2 | H | H | $C_2H_5$ | $OCH_3$ | O | 3 | |
| 2.19 | Cyclobutyl | 2 | H | H | $CH_3$ | $OC_2H_5$ | O | 3 | |
| 2.20 | Cyclobutyl | 2 | H | H | $OCH_3$ | $OCH_2CF_3$ | O | 3 | |
| 2.21 | Cyclopentyl | 2 | H | H | $OCH_3$ | $N(CH_3)_2$ | O | 3 | |
| 2.22 | Cyclopentyl | 2 | H | H | $OCH_3$ | $NH-CH_3$ | O | 3 | |
| 2.23 | Cyclopentyl | 2 | H | H | $OCH_3$ | Cyclopropyl | O | 3 | |

| | | | | | | | | | |
|---|---|---|---|---|---|---|---|---|---|
| 2.24 | Cyclopentyl | 2 | H | H | $OCH_3$ | $SCH_3$ | O | 3 | |
| 2.25 | Cyclopentyl | 2 | H | H | $OCH_3$ | $OCH_2CF_3$ | O | 3 | |
| 2.26 | Cyclopentyl | 2 | H | H | $OCH_3$ | $CH_2F$ | O | 3 | |
| 2.27 | Cyclopentyl | 2 | H | H | $CH_3$ | $CH_2Cl$ | O | 3 | |
| 2.28 | Cyclopentyl | 2 | H | H | $OCH_3$ | $CH_2Cl$ | O | 3 | |
| 2.29 | Cyclopentyl | 2 | H | H | $OCH_3$ | $CH_2OCH_3$ | O | 3 | |
| 2.30 | Cyclopentyl | 2 | H | H | $CH_3$ | $OCH_2CF_3$ | O | 3 | |
| 2.31 | Cyclopentyl | 2 | H | H | $CH_3$ | $CH_3$ | O | 3 | |
| 2.32 | Cyclopentyl | 2 | H | H | Cl | $CH_3$ | O | 3 | |
| 2.33 | 2-Cyclopentenyl | 2 | H | H | $CH_3$ | $OCH_3$ | O | 3 | |
| 2.34 | 2-Cyclopentenyl | 2 | H | H | $OCH_3$ | $OCH_3$ | O | 3 | |
| 2.35 | 2-Cyclopentenyl | 2 | H | H | $C_2H_5$ | $OCH_3$ | O | 3 | |
| 2.36 | 2-Cyclopentenyl | 2 | H | H | $CH_3$ | $OC_2H_5$ | O | 3 | |
| 2.37 | 2-Cyclopentenyl | 2 | H | H | $OCH_3$ | $OCH_2CF_3$ | O | 3 | |
| 2.38 | 2-Chlorcyclopentyl | 2 | H | H | $CH_3$ | $OCH_3$ | O | 3 | |
| 2.39 | 2-Chlorcyclopentyl | 2 | H | H | $OCH_3$ | $OCH_3$ | O | 3 | |
| 2.40 | 2-Chlorcyclopentyl | 2 | H | H | $C_2H_5$ | $OCH_3$ | O | 3 | |
| 2.41 | 2-Chlorcyclopentyl | 2 | H | H | $CH_3$ | $OC_2H_5$ | O | 3 | |
| 2.42 | 2-Chlorcyclopentyl | 2 | H | H | $OCH_3$ | $OCH_2CF_3$ | O | 3 | |
| 2.43 | 2-Fluorcyclopentyl | 2 | H | H | $CH_3$ | $OCH_3$ | O | 3 | |
| 2.44 | 2-Fluorcyclopentyl | 2 | H | H | $OCH_3$ | $OCH_3$ | O | 3 | |
| 2.45 | Cyclopentyl | 2 | 5-F | H | $CH_3$ | $OCH_3$ | O | 3 | |
| 2.46 | Cyclopentyl | 2 | 5-F | H | $OCH_3$ | $OCH_3$ | O | 3 | |
| 2.47 | Cyclopentyl | 2 | 5-Cl | H | $CH_3$ | $OCH_3$ | O | 3 | |
| 2.48 | Cyclopentyl | 2 | 5-Cl | H | $OCH_3$ | $OCH_3$ | O | 3 | |
| 2.49 | Cyclopentyl | 2 | 5-$OCH_3$ | H | $CH_3$ | $OCH_3$ | O | 3 | |
| 2.50 | Cyclopentyl | 2 | 5-$OCH_3$ | H | $OCH_3$ | $OCH_3$ | O | 3 | |
| 2.51 | Cyclopentyl | 2 | 5-$NO_2$ | H | $CH_3$ | $OCH_3$ | O | 3 | |
| 2.52 | Cyclopentyl | 2 | 5-$NO_2$ | H | $OCH_3$ | $OCH_3$ | O | 3 | |
| 2.53 | Cyclopentyl | 2 | 6-Cl | H | $CH_3$ | $OCH_3$ | O | 3 | |
| 2.54 | Cyclopentyl | 2 | 6-Cl | H | $OCH_3$ | $OCH_3$ | O | 3 | |
| 2.55 | Cyclopentyl | 2 | 6-$COOCH_3$ | H | $CH_3$ | $OCH_3$ | O | 3 | |
| 2.56 | Cyclopentyl | 2 | 6-$COOCH_3$ | H | $OCH_3$ | $OCH_3$ | O | 3 | |
| 2.57 | Cyclopentyl | 2 | H | $CH_3$ | $OCH_3$ | $OCH_3$ | O | 3 | |
| 2.58 | Cyclopentyl | 2 | H | H | $CH_3$ | $OCH_3$ | S | 3 | |
| 2.59 | Cyclopentyl | 2 | H | H | $OCH_3$ | $OCH_3$ | S | 3 | |
| 2.60 | Cyclopropylmethyl | 2 | H | H | $CH_3$ | $OCH_3$ | O | 3 | 165-167 |
| 2.61 | Cyclopropylmethyl | 2 | H | H | $OCH_3$ | $OCH_3$ | O | 3 | 164-165 |
| 2.62 | Cyclopropylmethyl | 2 | H | H | $C_2H_5$ | $OCH_3$ | O | 3 | |
| 2.63 | Cyclopropylmethyl | 2 | H | H | $CH_3$ | $OC_2H_5$ | O | 3 | |
| 2.64 | Cyclopropylmethyl | 2 | H | H | $OCH_3$ | $OCH_2CF_3$ | O | 3 | |
| 2.65 | 1-Methylcyclopropyl | 2 | H | H | $CH_3$ | $OCH_3$ | O | 3 | |
| 2.66 | 1-Methylcyclopropyl | 2 | H | H | $OCH_3$ | $OCH_3$ | O | 3 | |
| 2.67 | Cyclohexylmethyl | 2 | H | H | $CH_3$ | $OCH_3$ | O | 3 | |
| 2.68 | Cyclohexylmethyl | 2 | H | H | $OCH_3$ | $OCH_3$ | O | 3 | |
| 2.69 | Cyclopentylmethyl | 2 | H | H | $CH_3$ | $OCH_3$ | O | 3 | |
| 2.70 | Cyclopentylmethyl | 2 | H | H | $OCH_3$ | $OCH_3$ | O | 3 | |
| 2.71 | 2-Oxocyclopentyl | 2 | H | H | $OCH_3$ | $OCH_3$ | O | 3 | |
| 2.72 | 2-Oxocyclopentyl | 2 | H | H | $OCH_3$ | $OCH_3$ | O | 3 | |
| 2.73 | 2-Methoxycyclopenyl | 2 | H | H | $CH_3$ | $OCH_3$ | O | 3 | |
| 2.74 | 2-Methoxlcyclopentyl | 2 | H | H | $OCH_3$ | $OCH_3$ | O | 3 | |
| 2.75 | 2-Cyanocyclopentyl | 2 | H | H | $CH_3$ | $OCH_3$ | O | 3 | |
| 2.76 | 2-Cyanocyclopentyl | 2 | H | H | $OCH_3$ | $OCH_3$ | O | 3 | |
| 2.77 | Cyclopentyl | 3 | H | H | $CH_3$ | $OCH_3$ | O | 2 | |
| 2.78 | Cyclopentyl | 3 | H | H | $OCH_3$ | $OCH_3$ | O | 2 | |
| 2.79 | Cyclohexyl | 3 | H | H | $CH_3$ | $OCH_3$ | O | 2 | |
| 2.80 | Cyclohexyl | 3 | H | H | $OCH_3$ | $OCH_3$ | O | 2 | |
| 2.81 | 2-Cyclopentenyl | 3 | H | H | $CH_3$ | $OCH_3$ | O | 2 | |
| 2.82 | 2-Cyclopentenyl | 3 | H | H | $OCH_3$ | $OCH_3$ | O | 2 | |
| 2.83 | 2-Chlorcyclopentyl | 3 | H | H | $CH_3$ | $OCH_3$ | O | 2 | |
| 2.84 | 2-Chlorcyclopentyl | 3 | H | H | $OCH_3$ | $OCH_3$ | O | 2 | |
| 2.85 | Cyclopropylmethyl | 3 | H | H | $CH_3$ | $OCH_3$ | O | 2 | |
| 2.86 | Cyclopropylmethyl | 3 | H | H | $OCH_3$ | $OCH_3$ | O | 2 | |
| 2.87 | Cyclohexylmethyl | 3 | H | H | $CH_3$ | $OCH_3$ | O | 2 | |
| 2.88 | Cyclohexylmethyl | 3 | H | H | $OCH_3$ | $OCH_3$ | O | 2 | |

**Tabelle 3**:

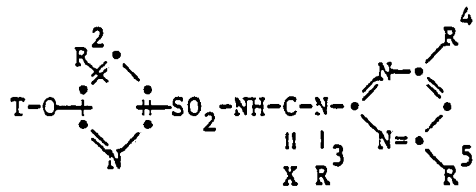

| Verb. No. | T | Stellung -O-T Rest | $R_2$ | $R_3R_4$ | $R_5$ | X | Stellung $-SO_2$-Rest | Smp. [°C] |
|---|---|---|---|---|---|---|---|---|
| 3.1 | Cyclopentyl | 2 | H | H $CH_3$ | $OCH_3$ | O | 3 | |
| 3.2 | Cyclopentyl | 2 | H | H $OCH_3$ | $OCH_3$ | O | 3 | 180-182 |
| 3.3 | Cyclopentyl | 2 | H | H Cl | $OCH_3$ | O | 3 | |
| 3.4 | Cyclopentyl | 2 | H | H $CH_3$ | $OC_2H_5$ | O | 3 | |
| 3.5 | Cyclopentyl | 2 | H | H $OCH_3$ | $OC_2H_5$ | O | 3 | |
| 3.6 | Cyclopentyl | 2 | H | H $CH_3$ | $OCHF_2$ | O | 3 | 161-163 |
| 3.7 | Cyclopentyl | 2 | H | H $OCH_3$ | $OCHF_2$ | O | 3 | |
| 3.8 | Cyclopentyl | 2 | H | H $OCHF_2$ | $OCHF_2$ | O | 3 | |
| 3.9 | Cyclopentyl | 2 | H | H $N(CH_3)_2$ | $OCHF_2$ | O | 3 | |
| 3.10 | Cyclopentyl | 2 | H | H Cl | $OCHF_2$ | O | 3 | |
| 3.11 | Cyclohexyl | 2 | H | H $CH_3$ | $OCH_3$ | O | 3 | |
| 3.12 | Cyclohexyl | 2 | H | H $OCH_3$ | $OCH_3$ | O | 3 | 196-199 |
| 3.13 | Cyclohexyl | 2 | H | H Cl | $OCH_3$ | O | 3 | |
| 3.14 | Cyclohexyl | 2 | H | H $CH_3$ | $OCHF_2$ | O | 3 | 169-171 |
| 3.15 | Cyclohexyl | 2 | H | H $OCH_3$ | $OCHF_2$ | O | 3 | |
| 3.16 | Cyclobutyl | 2 | H | H $CH_3$ | $OCH_3$ | O | 3 | |
| 3.17 | Cyclobutyl | 2 | H | H $OCH_3$ | $OCH_3$ | O | 3 | |
| 3.18 | Cyclobutyl | 2 | H | H Cl | $OCH_3$ | O | 3 | |
| 3.19 | Cyclobutyl | 2 | H | H $CH_3$ | $OCHF_2$ | O | 3 | |
| 3.20 | Cyclobutyl | 2 | H | H $OCH_3$ | $OCHF_2$ | O | 3 | |
| 3.21 | Cyclopentyl | 2 | H | H $OCH_3$ | $N(CH_3)_2$ | O | 3 | |
| 3.22 | Cyclopentyl | 2 | H | H $OCH_3$ | $NH-CH_3$ | O | 3 | |
| 3.23 | Cyclopentyl | 2 | H | H $OCH_3$ | Cyclopropyl | O | 3 | |
| 3.24 | Cyclopentyl | 2 | H | H $OCH_3$ | $SCH_3$ | O | 3 | |
| 3.25 | Cyclopentyl | 2 | H | H $OCH_3$ | $OCH_2CF_3$ | O | 3 | |
| 3.26 | Cyclopentyl | 2 | H | H $OCHF_2$ | $CH_2F$ | O | 3 | |
| 3.27 | Cyclopentyl | 2 | H | H $OCH_3$ | $CH_2F$ | O | 3 | |
| 3.28 | Cyclopentyl | 2 | H | H $OCH_3$ | $CH_2Cl$ | O | 3 | |
| 3.29 | Cyclopentyl | 2 | H | H $OCH_3$ | $CH_2OCH_3$ | O | 3 | |
| 3.30 | Cyclopentyl | 2 | H | H $CH_3$ | $OCH_2CF_3$ | O | 3 | |
| 3.31 | Cyclopentyl | 2 | H | H $CH_3$ | $CH_3$ | O | 3 | |
| 3.32 | Cyclopentyl | 2 | H | H Cl | $CH_3$ | O | 3 | |
| 3.33 | 2-Cyclopentenyl | 2 | H | H $CH_3$ | $OCH_3$ | O | 3 | |
| 3.34 | 2-Cyclopentenyl | 2 | H | H $OCH_3$ | $OCH_3$ | O | 3 | |
| 3.35 | 2-Cyclopentenyl | 2 | H | H Cl | $OCH_3$ | O | 3 | |
| 3.36 | 2-Cyclopentenyl | 2 | H | H $CH_3$ | $OCHF_2$ | O | 3 | |
| 3.37 | 2-Cyclopentenyl | 2 | H | H $OCH_3$ | $OCHF_2$ | O | 3 | |
| 3.38 | 2-Chlorcyclopentyl | 2 | H | H $CH_3$ | $OCH_3$ | O | 3 | |
| 3.39 | 2-Chlorcyclopentyl | 2 | H | H $OCH_3$ | $OCH_3$ | O | 3 | |
| 3.40 | 2-Chlorcyclopentyl | 2 | H | H Cl | $OCH_3$ | O | 3 | |
| 3.41 | 2-Chlorcyclopentyl | 2 | H | H $CH_3$ | $OCHF_2$ | O | 3 | |
| 3.42 | 2-Chlorcyclopentyl | 2 | H | H $OCH_3$ | $OCHF_2$ | O | 3 | |
| 3.43 | 2-Fluorcyclopentyl | 2 | H | H $CH_3$ | $OCH_3$ | O | 3 | |
| 3.44 | 2-Fluorcyclopentyl | 2 | H | H $OCH_3$ | $OCH_3$ | O | 3 | |
| 3.45 | Cyclopentyl | 2 | 5-F | H $CH_3$ | $OCH_3$ | O | 3 | |
| 3.46 | Cyclopentyl | 2 | 5-F | H $0CH_3$ | $0CH_3$ | O | 3 | |
| 3.47 | Cyclopentyl | 2 | 5-Cl | H $CH_3$ | $OCH_3$ | O | 3 | |
| 3.48 | Cyclopentyl | 2 | 5-Cl | H $OCH_3$ | $OCH_3$ | O | 3 | |
| 3.49 | Cyclopentyl | 2 | 5-$OCH_3$ | H $CH_3$ | $OCH_3$ | O | 3 | |
| 3.50 | Cyclopentyl | 2 | 5-$OCH_3$ | H $OCH_3$ | $OCH_3$ | O | 3 | |
| 3.51 | Cyclopentyl | 2 | 5-$NO_2$ | H $CH_3$ | $OCH_3$ | O | 3 | |
| 3.52 | Cyclopentyl | 2 | 5-$NO_2$ | H $OCH_3$ | $OCH_3$ | O | 3 | |

| | | | | | | | | | |
|---|---|---|---|---|---|---|---|---|---|
| 3.53 | Cyclopentyl | 2 | 6-Cl | H | $CH_3$ | $OCH_3$ | O | 3 | |
| 3.54 | Cyclopentyl | 2 | 6-Cl | H | $OCH_3$ | $OCH_3$ | O | 3 | |
| 3.55 | Cyclopentyl | 2 | 6-$COOCH_3$ | H | $CH_3$ | $OCH_3$ | O | 3 | |
| 3.56 | Cyclopentyl | 2 | 6-$COOCH_3$ | H | $OCH_3$ | $OCH_3$ | O | 3 | |
| 3.57 | Cyclopentyl | 2 | H | $CH_3$ | $OCH_3$ | $OCH_3$ | O | 3 | |
| 3.58 | Cyclopentyl | 2 | H | H | $CH_3$ | $OCH_3$ | S | 3 | |
| 3.59 | Cyclopentyl | 2 | H | H | $OCH_3$ | $OCH_3$ | S | 3 | |
| 3.60 | Cyclopropylmethyl | 2 | H | H | $CH_3$ | $OCH_3$ | O | 3 | |
| 3.61 | Cyclopropylmethyl | 2 | H | H | $OCH_3$ | $OCH_3$ | O | 3 | 167-16 |
| 3.62 | Cylopropylmethyl | 2 | H | H | Cl | $OCH_3$ | O | 3 | |
| 3.63 | Cyclopropylmethyl | 2 | H | H | $CH_3$ | $OCHF_2$ | O | 3 | 156-158 |
| 3.64 | Cyclopropylmethyl | 2 | H | H | $OCH_3$ | $OCHF_2$ | O | 3 | |
| 3.65 | 1-Methylcyclopropyl | 2 | H | H | $CH_3$ | $OCH_3$ | O | 3 | |
| 3.66 | 1-Methylcyclopropyl | 2 | H | H | $OCH_3$ | $OCH_3$ | O | 3 | |
| 3.67 | Cyclohexylmethyl | 2 | H | H | $CH_3$ | $OCH_3$ | O | 3 | |
| 3.68 | Cyclohexylmethyl | 2 | H | H | $OCH_3$ | $OCH_3$ | O | 3 | |
| 3.69 | Cyclopentylmethyl | 2 | H | H | $CH_3$ | $OCH_3$ | O | 3 | |
| 3.70 | Cyclopentylmethyl | 2 | H | H | $OCH_3$ | $OCH_3$ | O | 3 | |
| 3.71 | 2-Oxocyclopentyl | 2 | H | H | $CH_3$ | $OCH_3$ | O | 3 | |
| 3.72 | 2-Oxocyclopentyl | 2 | H | H | $OCH_3$ | $OCH_3$ | O | 3 | |
| 3.73 | 2-Methoxycyclopentyl | 2 | H | H | $CH_3$ | $OCH_3$ | O | 3 | |
| 3.74 | 2-Methoxycyclopentyl | 2 | H | H | $OCH_3$ | $OCH_3$ | O | 3 | |
| 3.75 | 2-Cyanocyclopentyl | 2 | H | H | $CH_3$ | $OCH_3$ | O | 3 | |
| 3.76 | 2-Cyanocyclopentyl | 2 | H | H | $OCH_3$ | $OCH_3$ | O | 3 | |
| 3.77 | Cyclopentyl | 3 | H | H | $CH_3$ | $OCH_3$ | O | 2 | |
| 3.78 | Cyclopentyl | 3 | H | H | $OCH_3$ | $OCH_3$ | O | 2 | |
| 3.79 | Cyclohexyl | 3 | H | H | $CH_3$ | $OCH_3$ | O | 2 | |
| 3.80 | Cyclohexyl | 3 | H | H | $OCH_3$ | $OCH_3$ | O | 2 | |
| 3.81 | 2-Cyclopentenyl | 3 | H | H | $CH_3$ | $OCH_3$ | O | 2 | |
| 3.82 | 2-Cyclopentyl | 3 | H | H | $OCH_3$ | $OCH_3$ | O | 2 | |
| 3.83 | 2-Chlorcyclopentyl | 3 | H | H | $CH_3$ | $OCH_3$ | O | 2 | |
| 3.84 | 2-Chlorcyclopentyl | 3 | H | H | $OCH_3$ | $OCH_3$ | O | 2 | |
| 3.85 | Cyclopropylmethyl | 3 | H | H | $CH_3$ | $OCH_3$ | O | 2 | |
| 3.86 | Cyclopropylmethyl | 3 | H | H | $OCH_3$ | $OCH_3$ | O | 2 | |
| 3.87 | Cyclopropylmethyl | 3 | H | H | $CH_3$ | $OCH_3$ | O | 2 | |
| 3.88 | Cyclopropylmethyl | 3 | H | H | $OCH_3$ | $OCH_3$ | O | 2 | |
| 3.89 | Cyclohexyl | 2 | H | H | $OCHF_2$ | $OCHF_2$ | O | 3 | |
| 3.90 | Cyclopentyl | 2 | H | H | $OCHF_2$ | $OCHF_2$ | O | 3 | |
| 3.91 | 2-Cyclopentenyl | 2 | H | H | $OCHF_2$ | $OCHF_2$ | O | 3 | |
| 3.92 | 2-Chlorcyclopentyl | 2 | H | H | $OCHF_2$ | $OCHF_2$ | O | 3 | |
| 3.93 | Cyclopropylmethyl | 2 | H | H | $OCHF_2$ | $OCHF_2$ | O | 3 | |

**Formulierungsbeispiele:**

**Beispiel F 1**: Formulierungsbeispiele für Wirkstoffe der Formel I (% = Gewichtsprozent)

| a) **Spritzpulver** | a) | b) | c) |
|---|---|---|---|
| Wirkstoff | 20 % | 50 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther /7-8 Mol ÄO) | - | 2 % | 2 % |
| Hochdisperse Kieselsäure | 5 % | 27 % | 27 % |
| Kaolin | 67 % | - | - |
| Natriumchlotid | - | - | 59,5 % |

Der Wirkstoff witd mit den Zusatzstoffen gut vermischt und in einet geeigneten Mühle gut vermahlen. Man erhält Spritzpulver, die sich mit Wasser zu Suspensionen jeder gewünschten Konzentration verdünnen lassen.

b) **Emulsions-Konzentrat**

| | a) | b) |
|---|---|---|
| Wirkstoff | 10 % | 1 % |
| Octylphenolpolyäthylenglykoläther (4-5 Mol ÄO) | 3 % | 3 % |
| Ca-Dodecylbenzolsulfonat | 3 % | 3 % |
| Ricinusölpolyglykoläther (36 Mol ÄO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

c) **Stäubemittel**

| | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) **Extruder Granulat**

| | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) **Umhüllungs-Granulat**

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) **Suspensions-Konzentrat**

| | a) | b) |
|---|---|---|
| Wirkstoff | 40 % | 5 % |
| Äthylenglykol | 10 % | 10 % |
| Nonylphenolpolyäthylenglykoläthet (15 Mol ÄO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in form einet 75 %igen wässrigen Emulsion | 0,8 % | 0,8 % |
| Wasset | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) **Salzlösung**

| | |
|---|---|
| Wirkstoff | 5 % |
| Isopropylamin | 1 % |
| Octylphenolpolyäthylenglykoläther (78 Mol ÄO) | 3 % |
| Wasser | 91 % |

**Biologische Beispiele:**

**Beispiel B 1**: Herbizidwirkung vor dem Auflaufen der Pflanzen

Kunststofftöpfe werden mit expandiertem Vermiculit (Dichte: 0,135 g/$cm^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während einer Keimphase von 4 bis 5 Tagen werden die Töpfe zur Erhöhung det örtlichen Luftfeuchtigkeit mit lichtdurchlässigem Material abgedeckt und mit deionisiertem Wasser gegossen. Nach dem 5.Tag wird dem Giesswasser 0,5 % eines handelsüblichen Flüssigdüngers (® Greenzit, ex Ciba-Geigy) zugesetzt. 12 Tage nach der Aussaat wird der Versuch ausgewertet und die Wirkung auf die Versuchspflanzen nach dem folgenden Maßstab bewertet:

1: Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

**pre-emergente Wirkung:**

Konzentration der Wirkstoffemulsion: 70,8 ppm

| **Testpflanze** Wirkstoff Nr. | Nasturtium | Stellaria | Agrostis | Digitaria |
|---|---|---|---|---|
| 2.1 | 2 | 2 | 2 | 2 |
| 2.2 | 2 | 2 | 2 | 2 |
| 2.11 | 2 | 2 | 3 | 2 |
| 2.12 | 2 | 2 | 2 | 3 |
| 2.60 | 2 | 2 | 2 | 2 |
| 2.61 | 2 | 2 | 2 | 2 |
| 3.2 | 1 | 1 | 1 | 1 |
| 3.6 | 2 | 2 | 2 | 2 |
| 3.12 | 2 | 2 | 2 | 2 |
| 3.14 | 2 | 2 | 2 | 2 |
| 3.61 | 2 | 2 | 2 | 2 |
| 3.63 | 2 | 2 | 2 | 2 |

**Beispiel B 2**: Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 600 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen), ohne dass dabei die Veesuchspflanzen geschädigt wurden.

**Beispiel B 3**: Wuchsregulierung an Sojabohnen

In Kunststoffbehältern mit einem Erde-Torf-Sandgemisch im Verhältnis 6 : 3 : 1 wetden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I

bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe det Formel I eine merkliche Erhöhung der Anzahl und des Gewichts der Schoten im Haupttrieb.

**Beispiel B 4**: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

**Beispiel B 5**: Wuchshemmung bei Gräsern

In Kunststoffschalen mit Erde-Torf-Sand-Gemisch (6 : 3 : 1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10-30 % im Vergleich zur unbehandelten Kontrolle.

**Patentansprüche** für die Vertragsstaaten BE, CH, DE, FR, GB, IT, LI, NL

1. N-Pyridinylsulfonyl-N'-pyrimidinyl- und -triazinylharnstoffe der Formel I

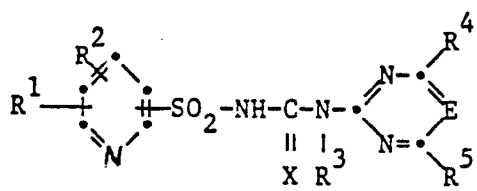

(I)

worin

$R^1$ einen Rest 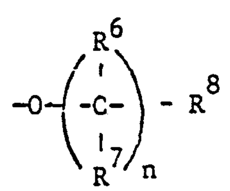

$R^2$ Wasserstoff, Halogen, Nitro, $C_1$-$C_3$-Alkyl, $C_1$-$C_3$-Alkoxy oder -$COOR^9$,
$R^3$ Wasserstoff, $C_1$-$C_3$-Alkyl oder $C_1$-$C_3$-Alkoxy,
$R^4$ und $R^5$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_2$-$C_4$-Alkoxyalkyl, $C_3$-$C_6$-Cycloalkyl oder -$NR^{10}R^{11}$,
$R^6$ Wasserstoff oder $C_1$-$C_3$-Alkyl,
$R^7$ Wasserstoff oder Methyl,
$R^8$ unsubstituiertes oder durch $C_1$-$C_3$-Alkyl oder Halogen substituiertes $C_4$-$C_6$-Cycloalkanoyl; unsubstituiertes oder ein- oder mehrfach durch $C_1$-C -Alkyl, $C_1$-$C_3$-Alkoxy, Halogen oder Cyan substituiertes $C_3$-$C_6$-Cycloalkyl;

oder unsubstituiertes oder durch $C_1$-$C_3$-Alkyl, Halogen oder Cyan substituiertes $C_5$-$C_6$-Cycloalkenyl,
$R^9$ $C_1$-$C_3$-Alkyl oder Allyl,
$R^{10}$ und $R^{11}$ unabhängig voneinander Wasserstoff oder $C_1$-$C_4$ -Alkyl,
n die Zahl Null oder eins,
X Sauerstoff oder Schwefel und
E Stickstoff oder die Methinbrücke bedeuten, sowie die Salze dieser Verbindungen.

2. Verbindungen gemäss Anspruch 1, dadurch gekennzeichnet, dass X für Sauerstoff steht.

3. Verbindungen gemäss Anspruch 1, dadurch gekennzeichnet, dass $R^2$ für Wasserstoff steht.

4. Verbindungen gemäss Anspruch 1, dadurch gekennzeichnet, dass $R^3$ für Wasserstoff steht.

5. Verbindungen gemäss Anspruch 1, dadurch gekennzeichnet, dass $R^1$ für $-O-R^8$ steht.

6. Verbindungen gemäss Anspruch 1, dadurch gekennzeichnet, dass $R^1$ für $-O-CHR^7$-Cyclopropyl steht.

7. Verbindungen gemäss Anspruch 5, dadurch gekennzeichnet, dass $R^8$ für $C_3$-$C_6$-Cycloalkyl, insbesondere Cyclopentyl oder Cyclohexyl, steht.

8. Verbindungen gemäss Anspruch 1, dadurch gekennzeichnet, dass $R^4$ und $R^5$ unabhängig voneinander für Halogen, $C_1$-$C_3$-Alkyl, $C_1$-$C_3$- Alkoxy, Di-$C_1$-$C_3$-alkylamino oder $C_1$-$C_3$-Halogenalkoxy stehen und zusammen höchstens 4 Kohlenstoffatome enthalten.

9. Verbindungen gemäss Anspruch 1, dadurch gekennzeichnet, dass X für Sauerstoff steht, $R^2$ und $R^3$ Wasserstoff bedeuten, $R^4$ und $R^5$ unabhängig voneinander für Halogen, $C_1$-$C_3$-Alkyl, $C_1$-$C_3$-Alkoxy, Di-$C_1$-$C_3$-alkylamino oder $C_1$-$C_3$-Halogenalkoxy stehen und zusammen höchstens 4 Kohlenstoffatome enthalten und $R^1$ für einen Rest $-O-R^8$ steht.

10. Verbindungen gemäss Anspruch 1, dadurch gekennzeichnet, dass X für Sauerstoff steht, $R^2$ und $R^3$ Wasserstoff bedeuten, $R^4$ und $R^5$ unabhängig voneinander für $C_1$-$C_3$-Alkyl, Halogen, $C_1$-$C_3$-Alkoxy, Di-$C_1$-$C_3$-alkylamino oder $C_1$-$C_3$-Halogenalkoxy stehen und zusammen höchstens 4 Kohlenstoffatome enthalten und $R^1$ für $C_3$-$C_6$-Cycloalkoxy steht.

11. N-(2-Cyclopentyloxypyridin-3-ylsulfonyl)-N'-(4-methoxy-6-methyl-1,3,5-triazin-2-yl)-harnstoff gemäss Anspruch 1.

12. N-(2-Cyclopropylmethoxypyridin-3-ylsulfonyl)-N'-(4-methoxy-6-methyl-1,3,5-triazin-2-yl)-harnstoff gemäss Anspruch 1.

13. Verfahren zur Herstellung der Verbindungen der Formel I nach Anspruch 1, dadurch gekennzeichnet, dass man ein substituiertes Pyridinylsulfonamid der Formel II

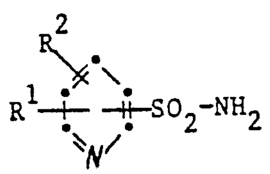

(II),

worin $R^1$ und $R^2$ die unter Formel I gegebene Bedeutung haben, in Gegenwart einer Base mit einem N-Pyrimidinyl- oder -triazinylcarbamat der Formel III

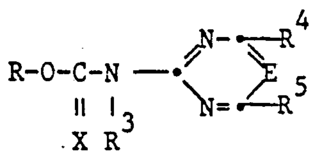

(III),

worin E, $R^3$, $R^4$, $R^5$, und X die unter Formel I gegebene Bedeutung haben und R für Phenyl, Alkyl oder substituiertes Phenyl, steht umgesetzt und gegebenenfalls in ein Salz überführt.

14. Verfahren zur Herstellung der Verbindungen der Formel I nach Anspruch 1, dadurch gekennzeichnet, dass man ein substituiertes N-Pyridinylsulfonylcarbamat der Formel IV

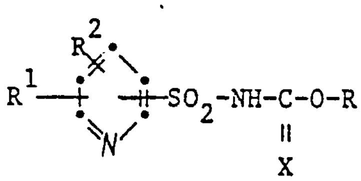

(IV),

worin $R^1$, $R^2$, X und Z die unter Formel I gegebene Bedeutung haben und R für Phenyl, Alkyl oder substituiertes Phenyl steht, mit einem Aminopyrimidin oder -triazin der Formel V

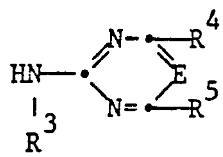

(V),

worin E, $R^3$, $R^4$ und $R^5$ die unter Formel I gegebene Bedeutung haben, umsetzt und gegebenenfalls in ein Salz überführt.

15. Verfahren zur Herstellung der Verbindungen der Formel I nach Anspruch 1, dadurch gekennzeichnet, dass man ein Sulfonylisocyanat der Formel VI

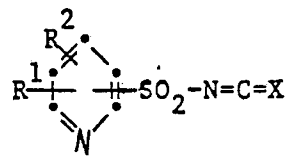

(VI),

worin $R^1$, $R^2$ und X die unter Formel I gegebene Bedeutung haben, mit einem Aminopyrimidin oder -triazin der im Anspruch 14 angegebenen Formel V umsetzt und gegebenenfalls in ein Salz überführt.

16. Verfahren zur Herstellung von Additionssalzen der Formel I gemäss einem der Ansprüche 13 bis 15, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

17. Ein herbizides und den Pflanzenwuchs hemmendes Mittel, dadurch gekennzeichnet, dass es neben Träger- und/oder anderen Zuschlagstoffen als Wirkstoff mindestens einen substituierten N-Arylsulfonyl-N'-pyrimidinyl- oder -triazinyl-harnstoff der Formel I, Anspruch 1, enthält.

18. Die Verwendung der Wirkstoffe der Formel I, gemäss Anspruch 1, oder sie enthaltender Mittel zur Bekämpfung unerwünschten Pflanzenwachstums.

19. Die Verwendung der Wirkstoffe der Formel I, gemäss Anspruch 1, oder sie enthaltender Mittel zur Hemmung des Pflanzenwachstums.

20. Die Verwendung der Wirkstoffe der Formel I, gemäss Anspruch 1, oder sie enthaltender Mittel zur Beeinflussung des Pflanzenwachstums zum Zweck der Ertragssteigerung.

21. Die Verwendung der Wirkstoffe der Formel I, oder sie enthaltender Mittel, gemäss Anspruch 18, zur selektiven pre- oder postemergenten Bekämpfung von Unkräutern in Nutzpflanzenkulturen.

22. Substituierte Pyridinylsulfonamide der Formel II

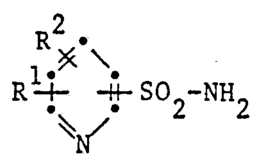

(II),

worin $R^1$ und $R^2$ die unter Formel I in Anspruch 1 angegebene Bedeutungen haben.

**Patentansprüche** für den Vertragsstaat AT

1. Ein herbizides und den Pflanzenwuchs regulierendes Mittel, dadurch gekennzeichnet, dass es neben Träger- und/oder anderen Zuschlagstoffen als Wirkstoff mindestens einen N-Pyridinylsulfonyl-N'-pyrimidinyl- und -triazinylharnstoff der Formel I

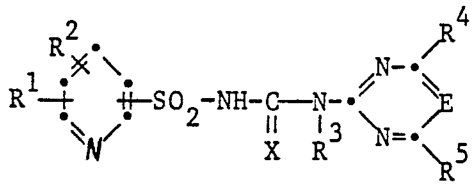

(I)

oder ein Salz davon enthält, worin

$R^1$ einen Rest 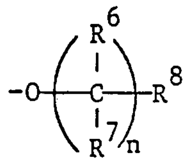

$R^2$ Wasserstoff, Halogen, Nitro, $C_1$-$C_3$-Alkyl, $C_1$-$C_3$-Alkoxy oder -$COOR^9$,
$R^3$ Wasserstoff, $C_1$-$C_3$-Alkyl oder $C_1$-$C_3$-Alkoxy,
$R^4$ und $R^5$ unabhängig voneinander Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_2$-$C_4$-Alkoxyalkyl, $C_3$-$C_6$-Cycloalkyl oder -$NR^{10}R^{11}$,
$R^6$ Wasserstoff oder $C_1$-$C_3$-Alkyl,
$R^7$ Wasserstoff oder Methyl,
$R^8$ unsubstituiertes oder durch $C_1$-$C_3$-Alkyl oder Halogen substituiertes $C_4$-$C_6$-Cycloalkanoyl; unsubstituiertes oder ein- oder mehrfach durch $C_1$-C-Alkyl, $C_1$-$C_3$-Alkoxy, Halogen oder Cyan substituiertes $C_3$-$C_6$-Cycloalkyl; oder unsubstituiertes oder durch $C_1$-$C_3$-Alkyl, Halogen oder Cyan substituiertes $C_5$-$C_6$-Cycloalkenyl,
$R^9$ $C_1$-$C_3$-Alkyl oder Allyl,
$R^{10}$ und $R^{11}$ unabhängig voneinander Wasserstoff oder $C_1$-$C_4$-Alkyl,
n die Zahl Null oder eins,
X Sauerstoff oder Schwefel und
E Stickstoff oder die Methinbrücke bedeuten.

2. Mittel gemäss Anspruch 1, dadurch gekennzeichnet, dass X für Sauerstoff steht.

3. Mittel gemäss Anspruch 1, dadurch gekennzeichnet, dass $R^2$ für Wasserstoff steht.

4. Mittel gemäss Anspruch 1, dadurch gekennzeichnet, dass $R^3$ für Wasserstoff steht.

5. Mittel gemäss Anspruch 1, dadurch gekennzeichnet, dass $R^1$ für -O-$R^8$ steht.

6. Mittel gemäss Anspruch 1, dadurch gekennzeichnet, dass $R^1$ für -O-$CHR^7$-Cyclopropyl steht.

7. Mittel gemäss Anspruch 5, dadurch gekennzeichnet, dass $R^8$ für $C_3$-$C_6$-Cycloalkyl, insbesondere Cyclopentyl oder Cyclohexyl, steht.

8. Mittel gemäss Anspruch 1, dadurch gekennzeichnet, dass $R^4$ und $R^5$ unabhängig voneinander für Halogen, $C_1$-$C_3$-Alkyl, $C_1$-$C_3$- Alkoxy, Di-$C_1$-$C_3$-alkylamino oder $C_1$-$C_3$-Halogenalkoxy stehen und zusammen höchstens 4 Kohlenstoffatome enthalten.

9. Mittel gemäss Anspruch 1, dadurch gekennzeichnet, dass X für Sauerstoff steht, $R^2$ und $R^3$ Wasserstoff bedeuten, $R^4$ und $R^5$ unabhängig voneinander für Halogen, $C_1$-$C_3$-Alkyl, $C_1$-$C_3$-Alkoxy, Di-$C_1$-$C_3$-alkylamino oder $C_1$-$C_3$-Halogenalkoxy stehen und zusammen höchstens 4 Kohlenstoffatome enthalten und Z für Stickstoff und $R^1$ für einen Rest -O-$R^8$ stehen.

10. Mittel gemäss Anspruch 1, dadurch gekennzeichnet, dass X für Sauerstoff steht, $R^2$ und $R^3$ Wasserstoff bedeuten, $R^4$ und $R^5$ unabhängig voneinander für $C_1$-$C_3$-Alkyl, Halogen, $C_1$-$C_3$-Alkoxy, Di-$C_1$-$C_3$-alkylamino oder $C_1$-$C_3$-Halogenalkoxy stehen und zusammen höchstens 4 Kohlenstoffatome enthalten und $R^1$ für $C_3$-$C_6$-Cycloalkoxy steht.

11. Mittel gemäss Anspruch 1, dadurch gekennzeichnet, dass es als Wirkstoff N-(2-Cyclopentyloxypyridin-3-ylsulfonyl)-N'-(4-methoxy-6-methyl-1,3,5-triazin-2-yl)-harnstoff enthält.

12. Mittel gemäss Anspruch 1, dadurch gekennzeichnet, dass es als Wirkstoff N-(2-Cyclopropylmethoxypyridin-3-ylsulfonyl)-N'-(4-methoxy-6-methyl-1,3,5-triazin-2-yl)-harnstoff enthält.

13. Verfahren zur Herstellung der Verbindungen der Formel I nach Anspruch 1, dadurch gekennzeichnet, dass man ein substituiertes Pyridinylsulfonamid der Formel II

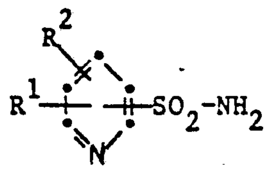

(II),

worin $R^1$ und $R^2$ die unter Formel I gegebene Bedeutung haben, in Gegenwart einer Base mit einem N-Pyrimidinyl- oder -triazinylcarbamat der Formel III

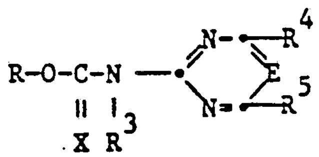

(III),

worin E, $R^3$, $R^4$, $R^5$ und X die unter Formel I gegebene Bedeutung haben und R für Phenyl, Alkyl oder substituiertes Phenyl steht, umgesetzt und gegebenenfalls im ein Salz überführt.

14. Verfahren zur Herstellung der Verbindungen der Formel I nach Anspruch 1, dadurch gekennzeichnet, dass man ein substituiertes N-Pyridinylsulfonylcarbamat der Formel IV

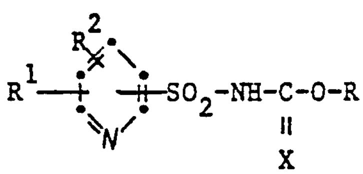

(IV),

worin $R^1$, $R^2$, und X die unter Formel I gegebene Bedeutung haben und R für Phenyl, Alkyl oder substituiertes Phenyl steht mit einem Aminopyrimidin oder -triazin der Formel V

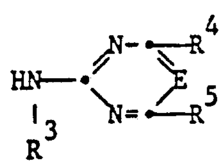

(V),

worin E, $R^3$, $R^4$ und $R^5$ die unter Formel I gegebene Bedeutung haben umsetzt und gegebenenfalls in ein Salz überführt.

15. Verfahren zur Herstellung der Verbindungen der Formel I, nach Anspruch 1, dadurch gekennzeichnet, dass man ein Sulfonylisocyanat der Formel VI

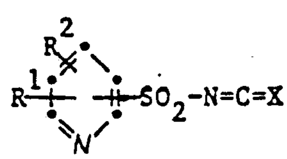

(VI),

worin $R^1$, $R^2$, X und Z die unter Formel I gegebene Bedeutung haben, mit einem Aminopyrimidin oder -triazin der im Anspruch 14 angegebenen Formel V umsetzt und gegebenenfalls in ein Salz überführt.

16. Verfahren zur Herstellung von Additionssalzen der Formel I gemäss einem der Ansprüche 13 bis 15 dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

17. Die Verwendung der Wirkstoffe der Formel I, gemäss Anspruch 1, oder sie enthaltender Mittel zur Bekämpfung unerwünschten Pflanzenwachstums.

18. Die Verwendung der Wirkstoffe der Formel I, gemäss Anspruch 1, oder sie enthaltender Mittel zur Hemmung des Pflanzenwachstums.

19. Die Verwendung der Wirkstoffe der Formel I, gemäss Anspruch 1, oder sie enthaltender Mittel zur Beeinflussung des Pflanzenwachstums zum Zweck der Ertragssteigerung.

20. Die Verwendung der Wirkstoffe der Formel I, oder sie enthaltender Mittel, gemäss Anspruch 17 zur selektiven pre- oder postemergenten Bekämpfung von Unkräutern in Nutzpflanzenkulturen.

**Claims** for the contracting states BE, CH, DE, FR, GB, IT, LI, NL

1. N-pyridinylsulfonyl-N'-pyrimidinylureas and N-pyridinylsulfonyl-N'-triazinylureas of the formula I

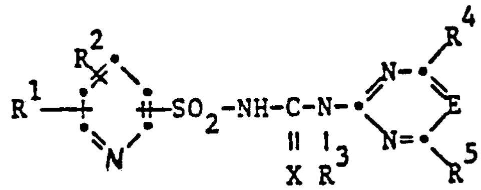

(I)

wherein
$R^1$ is a

wherein
$R^1$ is a

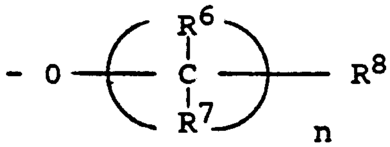

radical,

radical,

$R^2$ is hydrogen, halogen, nitro, $C_1$-$C_3$alkyl, C1-$C^3$alkoxy or -$COOR^9$,

$R^3$ is hydrogen, $C_1$-$C_3$alkyl or $C_1$-$C_3$alkoxy,

$R^4$ and $R^5$ are each independently of the other hydrogen, halogen $C_1$-$C_4$alkyl, $C_1$-$C_4$haloalkyl, $C_1$-$C_4$alkoxy, $C_1$-$C_4$haloalkoxy, $C_1$-$C_4$alkylthio, $C_1$-$C_4$haloalkylthio, $C_2$-$C_4$alkoxyalkyl, $C_3$-$C_6$cycloalkyl or -$NR^{10}R^{11}$,

$R^6$ is hydrogen or $C_1$-$C_3$alkyl,

$R^7$ is hydrogen or methyl,

$R^8$ is $C_4$-$C_6$cycloalkanoyl which is unsubstituted or substituted by $C_1$-$C_3$alkyl or halogen; $C_3$-$C_6$cycloalkyl which is unsubstituted or substituted by one or more members selected from the group consisting of $C_1$-C alkyl, $C_1$-$C_3$alkoxy, halogen or cyano; or is $C_5$-$C_6$cycloalkenyl which is unsubstituted or substituted by $C_1$-$C_3$alkyl, halogen or cyano,

$R^9$ is $C_1$-$C_3$alkyl or allyl,

$R^{10}$ and $R^{11}$ are each independently of the other hydrogen or $C_1$-$C_4$alkyl,

n is 0 or 1,

X is oxygen or sulfur, and

E is nitrogen or the methine bridge, and the salts of these compounds.

2. Compounds according to claim 1, characterised in that X is oxygen.

3. Compounds according to claim 1, characterised in that $R^2$ is hydrogen.

4. Compounds according to claim 1, characterised in that $R^3$ is hydrogen.

5. Compounds according to claim 1, characterised in that $R^1$ is -O-$R^8$.

6. Compounds according to claim 1, characterised in that $R^1$ is -O-$CHR^7$-cyclopropyl.

7. Compounds according to claim 5, characterised in that $R^8$ is $C_3$-$C_6$cycloalkyl, in particular cyclopentyl or cyclohexyl.

8. Compounds according to claim 1, characterised in that $R^4$ and $R^5$ are each independently of the other halogen, $C_1$-$C_3$alkyl, $C_1$-$C_3$alkoxy, di-$C_1$-$C_3$-alkylamino or $C_1$-$C_3$haloalkoxy, and together contain not more than 4 carbon atoms.

9. Compounds according to claim 1, characterised in that X is oxygen, $R^2$ and $R^3$ are hydrogen, $R^4$ and $R^5$ are each independently of the other halogen, $C_1$-$C_3$alkyl, $C_1$-$C_3$alkoxy, di-$C_1$-$C_3$-alkylamino or $C_1$-$C_3$haloalkoxy, and together contain not more that 4 carbon atoms, and $R^1$ is an -O-$R^8$ radical.

10. Compounds according to claim 1, characterised in that X is oxygen, $R^2$ and $R^3$ are hydrogen, $R^4$ and $R^5$ are each independently of the other $C_1$-$C_3$alkyl, halogen, $C_1$-$C_3$alkoxy, di-$C_1$-$C_3$-alkylamino or $C_1$-$C_3$haloalkoxy, and together contain not more than 4 carbon atoms, and $R^1$ is $C_3$-$C_6$cycloalkoxy.

11. N-(2-cyclopentyloxypyridin-3-ylsulfonyl)-N'-(4-methoxy-6-methyl-1,3,5-triazin-2-yl)urea according to claim 1.

12. N-(2-cyclopropylmethoxypyridin-3-ylsulfonyl)-N'-(4-methoxy-6-methyl-1,3,5-triazin-2-yl)urea according to claim 1.

13. A process for the preparation of compounds of formula I according to claim 1, characterised in that a substituted pyridinylsulfonamide of the formula II

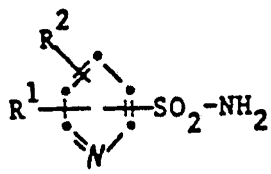

(II),

wherein $R^1$ and $R^2$ are as defined for formula I, is reacted with an N-pyrimidinylcarbamate or N-triazinylcarbamate of the formula III

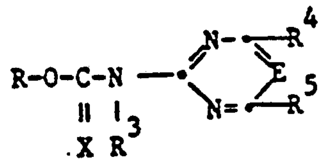

(III),

wherein E, $R^3$, $R^4$, $R^5$ and X are as defined for formula I and R is phenyl, alkyl or substituted phenyl, in the presence of a base, and, if desired, converted into a salt.

14. A process for the preparation of compounds of the formula I according to claim 1, characterised in that a substituted N-pyridinylsulfonylcarbamate of the formula IV

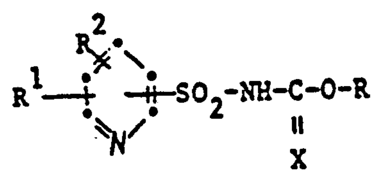

(IV),

wherein $R^1$, $R^2$, X and Z are as defined for formula I and R is phenyl, alkyl or substituted phenyl, is reacted with an aminopyrimidine or aminotriazine of the formula V

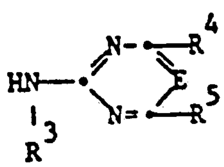

(V),

wherein E, $R^3$, $R^4$ and $R^5$ are as defined for formula I, and, if desired, converted into a salt.

15. A process for the preparation of compounds of formula I according to claim 1, characterised in that a sulfonylisocyanate of the formula VI

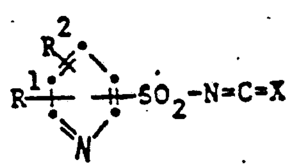

(VI),

wherein $R^1$, $R^2$ and X are as defined for formula I, is reacted with an aminopyrimidine or aminotriazine of the formula V as indicated in claim 14, and, if desired, converted into a salt.

16. A process for the preparation of addition salts of the formula I according to any one of claims 13 to 15, characterised in that a sulfonylurea of the formula I is reacted with an amine, an alkali metal hydroxide or alkaline earth metal hydroxide or with a quaternary ammonium base.

17. A herbicidal and plant growth-inhibiting composition characterised in that it contains as active ingredient at least one substituted N-arylsulfonyl-N'-pyrimidinylurea or N-arylsulfonyl-N'-triazinylurea of the formula I, claim 1, together with carriers and/or other adjuvants.

18. The use of the compounds of the formula I according to claim 1, or of compositions containing them, for controlling undesired plant growth.

19. The use of the compounds of the formula I according to claim 1, or of compositions containing them, for inhibiting plant growth.

20. The use of the compounds of the formula I according to claim 1, or of compositions containing them, for influencing plant growth for the purpose of increasing yield.

21. The use of the compounds of the formula I, or of compositions containing them, according to claim 18 for selectively controlling weeds pre- or post-emergence in crops of useful plants.

22. Substituted pyridinylsulfonamides of the formula II

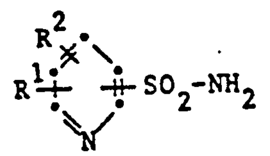 (II),

wherein $R^1$ and $R^2$ are as defined for formula I in claim 1.

**Claims** for the contracting state AT

1. A herbicidal and plant growth-regulating composition, characterised in that in addition to carriers and/or other adjuvants it contains as active ingredient at least one N-pyridinylsulfonyl-N'-pyrimidinylurea and N-pyridinylsulfonyl-N'-triazinylurea of the formula I

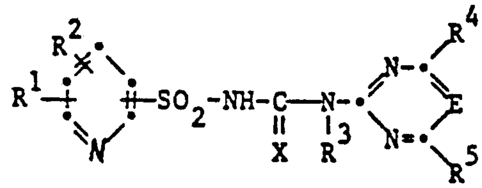

or a salt thereof, wherein

$R^1$ is a

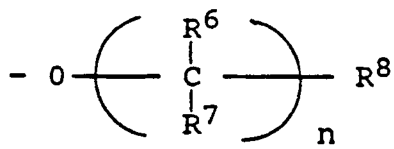 radical,

$R^2$ is hydrogen, halogen, nitro, $C_1$-$C_3$alkyl, $C_1$-$C_3$alkoxy or -$COOR^9$,

$R^3$ is hydrogen, $C_1$-$C_3$alkyl or $C_1$-$C_3$alkoxy,

$R^4$ and $R^5$ are each independently of the other hydrogen, halogen, $C_1$-$C_4$alkyl, $C_1$-$C_4$haloalkyl, $C_1$-$C_4$alkoxy, $C_1$-$C_4$haloalkoxy, $C_1$-$C_4$alkylthio, $C_1$-$C_4$haloalkylthio, $C_2$-$C_4$alkoxyalkyl, $C_3$-$C_6$cycloalkyl or -$NR^{10}R^{11}$,

$R^6$ is hydrogen or $C_1$-$C_3$alkyl,

$R^7$ is hydrogen or methyl,

$R^8$ is $C_4$-$C_6$cycloalkanoyl which is unsubstituted or substituted by $C_1$-$C_3$alkyl or halogen; $C_3$-$C_6$cycloalkyl which is unsubstituted or substituted by one or more members selected from the group consisting of $C_1$-C alkyl, $C_1$-$C_3$alkoxy, halogen or cyano; or is $C_5$-$C_6$cycloalkenyl which is unsubstituted or substituted by $C_1$-$C_3$alkyl, halogen or cyano,

$R^9$ is $C_1$-$C_3$alkyl or allyl,

$R^{10}$ and $R^{11}$ are each independently of the other hydrogen or $C^1$-$C_4$alkyl,

n is 0 or 1,

X is oxygen or sulfur, and

E is nitrogen or the methine bridge.

2. A composition according to claim 1, characterised in that X is oxygen.

3. A composition according to claim 1, characterised in that $R^2$ is hydrogen.

4. A composition according to claim 1, characterised in that $R^3$ is hydrogen.

5. A composition according to claim 1, characterised in that $R^1$ is -O-$R^8$.

6. A composition according to claim 1, characterised in that $R^1$ is -O-$CHR^7$-cyclopropyl.

7. A composition according to claim 5, characterised in that $R^8$ is $C_3$-$C_6$cycloalkyl, in particular cyclopentyl or cyclohexyl.

8. A composition according to claim 1, characterised in that $R^4$ and $R^5$ are each independently of the other halogen, $C_1$-$C_3$alkyl, $C_1$-$C_3$alkoxy, di-$C_1$-$C_3$-alkylamino or $C_1$-$C_3$haloalkoxy, and together contain not more than 4 carbon atoms.

9. A composition according to claim 1, characterised in that X is oxygen, $R^2$ and $R^3$ are hydrogen, $R^4$ and $R^5$ are each independently of the other halogen, $C_1$-$C_3$alkyl, $C_1$-$C_3$alkoxy, di-$C_1$-$C_3$-alkylamino or $C_1$-$C_3$haloalkoxy, and together contain not more that 4 carbon atoms, and Z is nitrogen and $R^1$ is an -O-$R^8$ radical.

10. A composition according to claim 1, characterised in that X is oxygen, $R^2$ and $R^3$ are hydrogen, $R^4$ and $R^5$ are each independently of the other $C_1$-$C_3$alkyl, halogen, $C_1$-$C_3$alkoxy, di-$C_1$-$C_3$-alkylamino or $C_1$-$C_3$haloalkoxy, and together contain not more than 4 carbon atoms, and $R^1$ is $C_3$-$C_6$cycloalkoxy.

11. A composition according to claim 1, characterised in that it contains as active ingredient N-(2-cyclopentyl-oxypyridin-3-ylsulfonyl)-N'-(4-methoxy-6-methyl-1,3,5-triazin-2-yl)urea.

12. A composition according to claim 1, characterised in that it contains as active ingredient N-(2-cyclopropyl-methoxypyridin-3-ylsulfonyl)-N'-(4-methoxy-6-methyl-1,3,5-triazin-2-yl)urea.

13. A process for the preparation of compounds of formula I according to claim 1, characterised in that a substituted pyridinylsulfonamide of the formula II

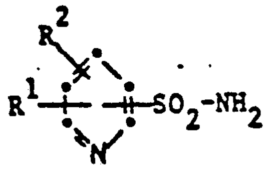

(II),

wherein $R^1$ and $R^2$ are as defined for formula I, is reacted with an N-pyrimidinylcarbamate or N-triazinylcarbamate of the formula III

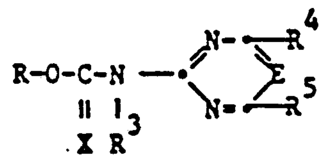

(III),

wherein E, $R^3$, $R^4$, $R^5$ and X are as defined for formula I and R is phenyl, alkyl or substituted phenyl, in the presence of a base, and, if desired, converted into a salt.

14. A process for the preparation of compounds of the formula I according to claim 1, characterised in that a substituted N-pyridinylsulfonylcarbamate of the formula IV

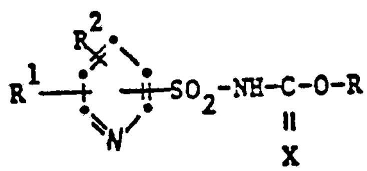

(IV),

wherein $R^1$, $R^2$ and X are as defined for formula I and R is phenyl, alkyl or substituted phenyl, is reacted with an aminopyrimidine or aminotriazine of the formula V

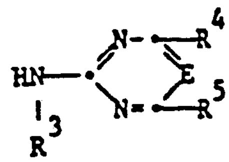

(V),

wherein E, $R^3$, $R^4$ and $R^5$ are as defined for formula I, and, if desired, converted into a salt.

15. A process for the preparation of compounds of formula I according to claim 1, characterised in that a sulfonylisocyanate of the formula VI

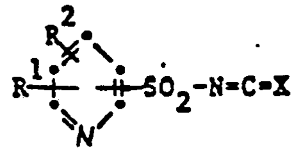

(VI),

wherein $R^1$, $R^2$, X and Z are as defined for formula I, is reacted with an aminopyrimidine or aminotriazine of the formula V as indicated in claim 14, and, if desired, converted into a salt.

16. A process for the preparation of addition salts of the formula I according to any one of claims 13 to 15, characterised in that a sulfonylurea of the formula I is reacted with an amine, an alkali metal hydroxide or alkaline earth metal hydroxide or with a quaternary ammonium base.

17. The use of the compounds of the formula I according to claim 1, or of compositions containing them, for controlling undesired plant growth.

18. The use of the compounds of the formula I according to claim 1, or of compositions containing them, for inhibiting plant growth.

19. The use of the compounds of the formula I according to claim 1, or of compositions containing them, for influencing plant growth for the purpose of increasing yield.

20. The use of the compounds of the formula I, or of compositions containing them, according to claim 17 for selectively controlling weeds pre- or post-emergence in crops of useful plants.

**Revendications** pour les Etats contractants BE, CH, DE, FR, GB, IT, LI, NL

1. Pyridinylsulfonyl-N'-pyrimidinyl- et -triazinylurée de formule I

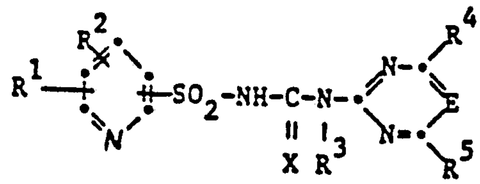

(I)

où

$R^1$ représente un radical 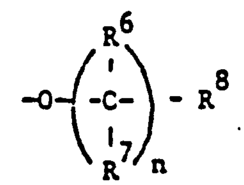

$R^2$ représente un hydrogène, halogène, nitro, alcoyle en $C_1$-$C_3$, alcoxy en $C_1C_3$ ou -$COOR^9$,

$R^3$ représente un hydrogène, alcoyle en $C_1$-$C_3$ ou alcoxy en $C_1$-$C_3$,

$R^4$ et $R^5$ représentent indépendamment l'un de l'autre un hydrogène, halogène, alcoyle en $C_1$-$C_4$, halogenalcoyle en $C_1$-$C_4$, alcoxy en $C_1$-$C_4$, halogenalcoylthio en $C_1$-$C_4$, alcoxyalcoyle en $C_2$-$C_4$, cycloalcoyle en $C_3$-$C_6$ ou -$NR^{10}R^{11}$,

$R^6$ représente un hydrogène ou un alcoyle en $C_1$-$C_3$,

$R^7$ représente un hydrogène ou un méthyle,

$R^8$ représente un cycloalcanoyle en $C_4$-$C_6$ non substitué ou substitué par un alcoyle en $C_1$-$C_3$ ou un halogène; un cycloalcoyle en $C_3$-$C_6$ non substitué ou mono ou polysubstitué par un alcoyle en $C_1$-$C_3$, alcoxy en $C_1$-$C_3$, halogène ou cyano; ou un cycloalcényle en $C_5$-$C_6$, non substitué ou substitué par un alcoyle en $C_1$-$C_3$, halogène ou cyano,

$R^9$ représente un alcoyle en $C_1$-$C_3$ ou un allyle,

$R^{10}$ et $R^{11}$ représentent indépendamment l'un de l'autre un hydrogène ou un alcoyle en $C_1$-$C_4$,

n représente le nombre zéro ou un,

X représente un oxygène ou un soufre et
E représente un azote ou le pont méthine ainsi que les sels de ces composés.

2. Composés selon la revendication 1, caractérisés en ce que X représente un oxygène.

3. Composés selon la revendication 1, caractérisés en ce que $R^2$ représente un hydrogène.

4. Composés selon la revendication 1, caractérisés en ce que $R^3$ représente un hydrogène.

5. Composés selon la revendication 1, caractérisés en ce que $R^1$ représente -O-$R^8$.

6. Composés selon la revendication 1, caractérisés en ce que $R^1$ représente -O-CH$R^7$-cyclopropyle.

7. Composés selon la revendication 5, caractérisés en ce que $R^8$ represente un cycloalcoyle en $C_3$-$C_6$, en particulier cyclopentyle ou cyclohexyle.

8. Composés selon la revendication 1, caractérisés en ce que $R^4$ et $R^5$ représentent indépendamment l'un de l'autre un halogène, alcoyle en $C_1$-$C_3$, alcoxy en $C_1$-$C_3$, di-alcoylamino en $C_1$-$C_3$ ou halogenalcoxy en $C_1$-$C_3$ et contiennent ensemble au plus 4 atomes de carbone.

9. Composés selon la revendication 1, caractérisés en ce que X représente un oxygène, $R^2$ et $R^3$ représentent un hydrogène, $R^4$ et $R^5$ représentent indépendamment l'un de l'autre un halogène, alcoyle en $C_1$-$C_3$, alcoxy en $C_1$-$C_3$, di-alcoylamino en $C_1$-$C_3$ ou halogenalcoxy en $C_1$-$C_3$ qui contiennent ensemble au plus 4 atomes de carbone et $R^1$ représente un radical -O-$R^8$.

10. Composés selon la revendication 1, caractérisés en ce que X représente un oxygène, $R^2$ et $R^3$ un hydrogène, $R^4$ et $R^5$ indépendamment l'un de l'autre un alcoyle en $C_1$-$C_3$, halogène, alcoxy en $C_1$-$C_3$, di-alcoylamino en $C_1$-$C_3$ ou halogenalcoxy en $C_1$-$C_3$ et contiennent ensemble au plus 4 atomes de carbone et $R^1$ représente un cycloalcoxy en $C_3$-$C_6$.

11. N-(2-cyclopentyloxypyridin-3-ylsulfonyl)-N'-(4-méthoxy-6-méthyl-1,3,5-triazin-2-yl)-urée, selon la revendication 1.

12. N-(2-cyclopropylméthoxypyridin-3-ylsulfonyl)-N'-(4-méthoxy-6-méthyl-1,3,5-triazin-2-yl)-urée, selon la revendication 1.

13. Procédés de préparation des composés de formule 1, selon la revendication I, caractérisés en ce qu'on fait réagir un pyridinylsulfonamide substitué de formule II

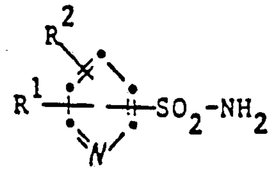

(II),

où $R^1$ et $R^2$ ont la signification donnée sous la formule I, en présence d'une base, avec un N-pyrimidinyle- ou -triazinylcarbamate de formule III

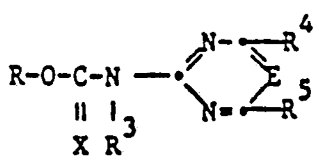

(III),

où E, $R^3$, $R^4$, $R^5$ et X ont la signification donnée sous la formule I et R représente un phényle, alcoyle ou phényle substitué et éventuellement en ce qu'on le transforme en un sel.

14. Procédés de préparation des composés de formule I selon la revendication 1, caractérisés en ce qu'on fait réagir un N-pyridinylsulfonylcarbamate substitué de fomule IV

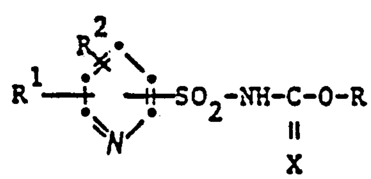

(IV),

où $R^1$, R, X et Z ont la signification donnée sont la formule I et R représente un phényle, alcoyle ou phényle substitué, avec une aminopyrimidine ou -triazine de formule V

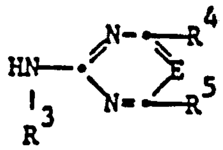

(V),

où E, $R^3$, et $R^5$ ont la signification donnée sous la formule I et éventuellement en ce qu'on le transforme en un sel.

15. Procédés de préparation de formule I, selon la revendication 1, caractérisés en ce qu'on fait réagir un sulfonylisocyanate de formule VI

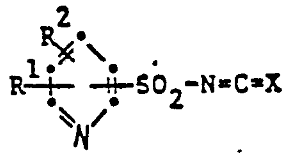

(VI),

où $R^1$, $R^2$ et X ont la signification donnée sous la formule I, avec une aminopyrimidine ou -triazine de formule V donnée dans la revendication 14 et éventuellement en ce qu'on le transforme en un seul point.

16. Procédés de préparation de sels d'addition d'acides de formule I selon l'une des revendications 13 à 15, caractérisés en ce qu'on fait réagir une sulfonylurée de formule I avec une amine, un hydroxyde de métal alcalin ou alcalino-terreux ou une base d'ammonium quaternaire.

17. Agent herbicide et inhibant la croissance des plantes caractérisé en ce qu'il contient outre des supports et/ou autres additifs, comme matières actives au moins une N-Arylsulfonyl-N'-pyrimidinyl- ou -triazinyl-urée substituée de formule I, de la revendication 1.

18. Application des matières actives de formule I selon la revendication 1 ou des agents qui les contiennent à la lutte contre la croissance végétale indésirable.

19. Application des matières actives de formule I, selon la revendication 1, ou des agents qui les contiennent à l'inhibition de la croissance végétale.

20. Application des matières actives de formule I, selon la revendication 1, ou des agents qui les contiennent pour influer sur la croissance des plantes dans le but d'accroître le rendement.

21. Application des matières actives de formule I, ou des agents qui les contiennent, selon la revendication 18, à la lutte sélective en pré- ou en post- levée contre les mauvaises herbes dans les cultures de plantes utiles.

22. Pyridinylsulfonamide substitué de formule II

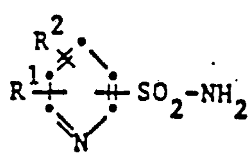

(II ),

où $R^1$ et $R^2$ ont les significations données sous la formule I dans la revendication 1.

**Revendications** pour l'Etat contractant AT

1. Agent herbicide et de régulation de la croissance des plantes, caractérisé en ce qu'il contient outre des supports et/ou autres additifs, comme matière active, au moins une N-pyridinylsulfonyl-N'-pyrimidinyl- et -triazinylurée de formule I

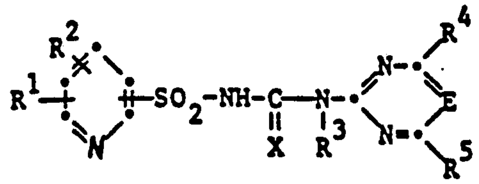

(I)

ou un de ses sels, où

$R^1$ représente un radical 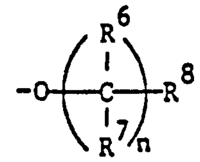

$R^2$ représente un hydrogène, halogène, nitro, alcoyle en $C_1$-$C_3$, alcoxy en $C_1$-$C_3$ ou $-COOR^9$,
$R^3$ représente un hydrogène, alcoyle en $C_1$-$C_3$ ou alcoxy en $C_1$-$C_3$,
$R^4$ et $R^5$ représentent indépendamment l'un de l'autre un hydrogène, halogène, alcoyle en $C_1$-$C_4$, halogenalcoyle en $C_1$-$C_4$, alcoxy en $C_1$-$C_4$, halogenalcoxy en $C_1$-$C_4$, alcoylthio en $C_1$-$C_4$, halogenalcoylthio en $C_1$-$C_4$, alcoxyalcoyle en $C_2$-$C_4$, cycloalcoyle en $C_3$-$C_6$ ou $-NR^{10}R^{11}$
$R^6$ représente un hydrogène ou un alcoyle en $C_1$-$C_3$,
$R^7$ represente un hydrogène ou un méthyle,
$R^8$ représente un cycloalcanoyle en $C_4$-$C_6$ non substitué ou substitué par un alcoyle en $C_1$-$C_3$, ou un halogène; un cycloalcoyle en $C_3$-$C_6$ non substitué ou mono ou polysubstitué par un alcoyle en $C_1$-$C_3$, alcoxy en $C_1$-$C_3$, halogène ou ou cyano; ou un cycloalcényle en $C_5$-$C_6$, non substitué ou substitué par un alcoyle en $C_1$-$C_3$, halogène ou cyano,
$R^9$ représente un alcoyle en $C_1$-$C_3$ ou un allyle,
$R^{10}$ et $R^{11}$ représentent indépendamment l'un de l'autre un hydrogène ou un alcoyle en $C_1$-$C_4$,
n représente le nombre Zéro ou un,
X représente un oxygène ou un soufre et
E représente un azote ou le pont méthine, ainsi que les sels de ces composés.

2. Agent selon la revendication 1, caractérisé en ce que X représente un oxygène.

3. Agent selon la revendication 1, caractérisé en ce que $R^2$ représente un hydrogène.

4. Agent selon la revendication 1, caractérisé en ce que $R^3$ représente un hydrogène.

5. Agent selon la revendication 1, caractérisé en ce que $R^1$ représente $-O-R^8$.

6. Agent selon la revendication 1, caractérisé en ce que $R^1$ représente-O-$CHR^7$-cyclopropyle.

7. Agent selon la revendication 5, caractérisé en ce que $R^8$ représente un cycloalcoyle en $C_3$-$C_6$, en particulier un cyclopentyle ou un cyclohexyle.

8. Agent selon la revendication 1, caractérisé en ce que $R^4$ et $R^5$ représentent indépendamment l'un de l'autre un halogène, alcoyle en $C_1$-$C_3$, alcoxy en $C_1$-$C_3$, di-alcoylamino en $C_1$-$C_3$ ou halogenalcoxy en $C_1$-$C_3$ et contiennent ensemble au plus 4 atomes de carbone.

9. Agent selon la revendication 1, caractérisé en ce que X représente un oxygène, $R^2$ et $R^3$ représentent un hydrogène, $R^4$ et $R^5$ représentent indépendamment l'un de l'autre un halogène, alcoyle en $C_1$-$C_3$, alcoxy en $C_1$-$C_3$, di-alcoylamino en $C_1$-$C_3$ ou halogenalcoxy en $C_1$-$C_3$ et contiennent ensemble au plus 4 atomes de carbone et Z représente un azote et $R^1$ un radical $-O-R^8$.

10. Agent selon la revendication 1, caractérisé en ce que X représente un oxygène, $R^2$ et $R^3$ représentent un hydrogène, $R^4$ et $R^5$ représentent indépendamment l'un de l'autre un alcoyle en $C_1$-$C_3$, un halogène, un alcoxy en $C_1$-$C_3$, un di- alcoylamino en $C_1$-$C_3$ ou un halogenalcoxy en $C_1$-$C_3$ et contiennent ensemble au plus 4 atomes de carbone et $R^1$ représente un cycloalcoxy en $C_3$-$C_6$.

11. Agent selon la revendication 1, caractérisé en ce qu'il contient comme matière active la N-(2-cyclopentyloxypyridin-3-ylsulfonyl)-N4-(4-méthoxy-6-méthyl-1,3,5-triazin-2-yl)-urée.

12. Agent selon la revendication 1, caractérisé en ce qu'il contient comme matière active la N-(2-cyclopropylméthoxy pyridin-3-ylsulfonyl)-N'-(4méthoxy-6-méthyl-1,3,5-triazin-2-yl)-urée.

13. Procédé de préparation des composés de formule I selon la revendication 1, caractérisé en ce qu'on fait réagir un pyridinylsulfonamide de formule II

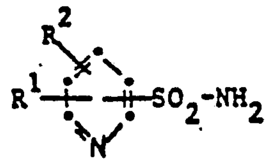 (II),

où $R^1$ et $R^2$ ont la signification donnée sous la formule I, en présence d'une base avec un N-pyrimidinyle- ou -triazinylcarbamate de formule III

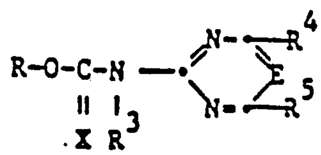 (III),

où E, $R^3$, $R^4$, $R^5$ et X ont la signification donnée sous la formule I et R représente un phényle, alcoyle ou phényle substitué et éventuellement en ce qu'on le transforme en un sel.

14. Procédé de préparation des composés de formule I, selon la revendication 1, caractérisé en ce qu'on fait réagir un N-pyridinylsulfonylcarbamate substitué de formule IV

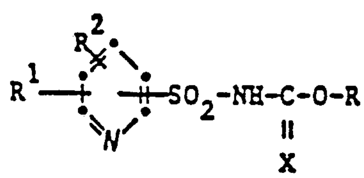 (IV),

où $R^1$, $R^2$, et X ont la signification donnée sous la formule I, et R représente un phényle, alcoyle ou phényle substitué, avec une mainopyrimidine ou -triazine de formule V

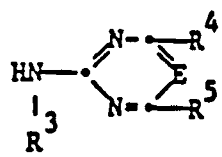 (V),

où E, $R^3$, $R^4$, et $R^5$ ont la signification donnée sous la formule I, et éventuellement en ce qu'on le transforme en un sel.

15. Procédé de préparation des composés de formule I, selon la revendication 1, caractérisé en ce qu'on fait réagir un sulfonylisocyanate de formule VI

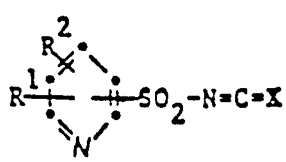 (VI),

où $R^1$, $R^2$, X et Z ont la signification donnée sous la formule I, avec une aminopyrimidine ou -triazine de formule V donnée dans la revendication 14 et éventuellement en ce qu'on le transforme en un sel.

16. Procédé de sels d'addition d'acides de formule I, selon l'une des revendications 13 à 15, caractérisé en ce qu'on fait réagir une sulfonylurée de formule I avec une amine, un hydroxyde de métal alcalin ou de métal alcalino-terreux ou une base d'ammonium quaternaire.

17. Application des matières actives de formule I selon la revendication 1 ou des agents qui les contiennent, à la lutte contre la croissance végétale indésirable.

18. Application des matières actives de formule I, selon la revendication 1, ou des agents qui les contiennent à l'inhibition de la croissance végétale.

19. Application des matières actives de formule I, selon la revendication I, ou des agents qui les contiennent pour influer sur la croissance végétale, dans le but d'accroître le rendement.

20. Application des matières actives de formule I ou des agents qui les contiennent selon la revendication 17, à la lutte sélective en pré- ou en post- levée contre la mauvaises herbes dans les cultures de plantes utiles.